# EUROPEAN PATENT APPLICATION

(11) **EP 2 583 744 A1**
(43) Date of publication of application: **24.04.2013**
(21) Application number: 12180228.4
(22) Date of filing: 29.03.2007
(51) Int. Cl.: B01D 61/14, B01D 61/16, C12M 1/12, C12M 1/16, B01D 61/20

(54) **Permeate product of tangential flow filtration process**

(30) Priority: 31.03.2006 US 788125 P
(62) Divisional of application: 07754371.8
(71) Applicant: Genencor International, Inc., Palo Alto, CA 94304 (US)
(72) Inventor: Dibel, Kevin, Palo Alto, CA California 94304 (US); Fong, Robin, Palo Alto, CA California 94304 (US); Heng, Meng, Hong, Palo Alto, CA California 94304 (US); Rozeboom, Glenn, Palo Alto, CA California 94304 (US)
(74) Representative: Andrews, Robert

(57) **Abstract**

The invention relates to apparatuses, machines, systems and methods for the recovery and purification of proteins, peptides, nucleic acids, biologically produced polymers and other compounds from aqueous fluids. The aqueous fluids can comprise enzyme concentrates and or a fermentation broth with or without cells or other starting material. The fermentation broth can be produced by fermentations of fungal, yeast, bacterial, mammalian, insect or plant cells.

## Description

### CROSS-REFERENCE TO RELATED APPLICATIONS

This application claims benefit of priority to U.S. Provisional Application No. 60/788,125, filed March 31, 2006, which is hereby incorporated by reference in its entirety.

### INTRODUCTION

The section headings used herein are solely for organization purposes and are not to be construed as limiting the subject matter described in any way.

The present invention relates to apparatuses, systems and methods for the separation, recovery, and/or purification of proteins, peptides, nucleic acids, biologically produced polymers and other compounds from aqueous fluids. The aqueous fluids can comprise a fermentation broth with or without cells or other starting material. The fermentation broth can be produced by fermentations of fungal, yeast, bacterial, mammalian, insect or plant cells.

### BACKGROUND

Microfiltration has been used for separation of compounds in biological broths or other liquids. The beverage industry has employed microfiltration to clarify beer and wine and in the dairy industry microfiltration can be used for processing of, for example, cheese whey or milk. Microfiltration has also recently been applied to the biotechnology industry, albeit somewhat more sparingly, for product separation and purification. An example is provided in the U.S. Patent Application No. US 2004/0167320 A1, Couto et al.*,* in which the processing of a solution through tangential flow filtration is discussed that can enhance clarification, concentration, and fractionation of desired molecules from a feedstream.

U.S. Patent Application No. US 20020020668 A1, Laustsen et al.*,* discusses a microfiltration process of a fermentation-derived product comprising adding activated carbon to a solution of the fermentation-derived product prior to or during the microfiltration process at a microfiltration process temperature of from 25° C to 65° C. In WO2004/029076, Christensen et al., discusses microfiltration processes comprising elevated temperatures.

In addition, information on microfiltration can be found in the scientific literature. Examples include, but are not restricted to, V. Chen et al., Journal of Membrane Science 125 (1997) 109-122; and L. Palacio, et al., Chemical Engineering (2003) 35-41.

A major drawback of various technologies is the requirement for repeated addition of raw materials to accomplish the filtration or separation. In the case of Rotary Drum Vacuum Filter (RDVF) and filter/membrane press, a filter aid such as diatomaceous earth or dicalite can be used to facilitate the separation of particles, and centrifugation can use differential particle density with flocculation aids (salts or charged polymers to increase particle size) to create the separation. This can increase the cost of production significantly, and these materials may have to be tailored to provide a specific maximum particle size that is allowed to pass.

Some processes can be exposed to the environment and increase the risk of contamination of the product as well as risk to operators from exposure to filter aids, raw broths, and products. Allergenic or other adverse reactions in exposed individuals may occur. Thus, environmental engineering controls may need to be designed and installed. Further, separated protein fractions produced by these technologies can have a much larger particle size distribution, and lower purity than those produced by the microfiltration process described herein. Descriptions of the aforementioned methods can be found in the scientific literature such as W. Aehle, ed.: Enzymes in industry: Production and Applications. Weinheim, F.R.D., New York, 2004, 1-12.

Column chromatography is a technology which can be used for biological broth purification. This process can provide a highly purified product, however, the production economics are typically orders of magnitude higher than for the methods described above and chromatography cannot handle particulate matter without major adjustments of the process, such as expanded bed columns. Expanded bed has not been widely adapted in industries that require high throughput and low cost.

As previously noted, microfiltration can be a method for biological broth clarification. It can also be used further down the process cascade as a method for removing particulates. Microfiltration can be applied in Tangential Flow Filtration mode (TFF) also known as Crossflow Filtration, in which the broth is pumped in a direction parallel to the membrane surface and a constant flow is maintained. A number of variables are important in tangential flow processes such as, membrane type, equipment design, solution properties, temperature, and operating conditions. Two operating parameters can be transmembrane pressure (TMP) and the crossflow velocity (CF). The transmembrane pressure is the force that pushes select components through the pores of the filter. The crossflow velocity is the flow rate of the liquid along the membrane surface. It provides the force that sweeps larger molecules and particulate matter away from the membrane surface. Such components can be organic or inorganic, dissolved polymers or particulates such as cells or cell debris. These components can foul the membrane thereby reducing the effectiveness of the process. In the TFF process a fluid feedstream is pumped from the feed vessel along the membrane surface (crossflow) in the filter and returned into the vessel as the retentate. Backpressure applied to the retentate tube by a valve or a clamp creates transmembrane pressure which drives the liquid and with it components smaller than the membrane pores creating a filtrate or permeate. In presently practiced microfiltration methods, the primary objective for the successful implementation of a TFF protocol is to optimize the TMP and CF so that a sample can be filtered effectively with high specificity without creating a membrane-fouling gel layer. The TMP can be adjusted throughout the filtration, or can be lowered during the concentration step to retain selectivity at higher concentrations. In contrast to ultrafiltration, in which the formation of a boundary layer can be desired to provide uniform pore sizes to only allow compounds within a specific molecular weight cut off to pass through, microfiltration is commonly operated at low TMP to avoid significant gel or boundary layer formation. Fouling layer formation can cause decreased passage of the desired compounds.

Embodiments of the inventions described herein can use, *inter alia,* TFF in a continuous or batch flow system that can be adapted for obtaining differing, selectable levels of purity and yields, depending on one's needs.

### SUMMARY

According to various embodiments, a multistage filtration system or apparatus is provided for producing differentiable filtration products in a facile and efficient manner.

According to various embodiments, a filtration is provided having a plurality of fluidly-interconnected filtration modules including a first module and multiple subsequent modules, wherein each module is configured to route received feed material and diluent adjacent a filter to provide permeate and retentate. The first module receives feed at an inlet side from outside the system and the subsequent modules receive retentate from a preceding module within the system as feed material. At least one of the modules has a permeate withdrawal flow line for withdrawing permeate from the system. A plurality of the modules have a permeate flow line configured for returning permeate back to an inlet side of a same or a preceding module within the system. This system permits enhanced control of collections and purifications of permeate products, retentate products, or both, being isolated by the system. Each filter can comprise an ultrafiltration membrane or another type of ultra- or micro-filtration component. The filtration system can be a tangential flow filtration (TFF) system.

According to various embodiments, the filtration system exemplified above can include first and subsequent modules that each have a permeate line for recycling permeate to the inlet side of the same module. The modules subsequent to the first module can further have a permeate line for backfeeding permeate to the inlet side of a preceding module within the system. Blending control can be provided by combining withdrawn permeate flows of two or more of the modules to provide a product having a targeted overall purity.

According to various embodiments, the filtration system has a control system that can provide independent adjustment of one of product yield, product purity, net permeation rate, and overall flux while maintaining the three other variables approximately constant. The configuration of the system exemplified herein allows it to be amenable to such independent control over these operational parameters. Conventional TFF machines and processes such as identified herein do not have multiple permeate streams (i.e., recycle, backfeed, withdrawal) at the filtration modules as provided in various embodiments of this present invention, nor the ability to control them independently. It is these multiple permeate streams and control of the above operating parameters that can provide more precise and predictable control of process parameters and product quality in various embodiments of the present invention. As shown by examples described herein, products produced by the system and process of the present invention have improved purity and other superior characteristics as compared to products produced by traditional filtration systems (e.g. RVDF) when used on similar feed materials.

According to other various embodiments, the filtration system comprises a first module including a permeate line for recycling permeate to the inlet side of the first module, and the modules subsequent to the first module each have a permeate line for recycling permeate to the inlet side of the same module and a permeate line for backfeeding permeate to the inlet side of a directly preceding module within the system, and has a heat exchange system to improve operating temperature control within the system. The heat exchange system includes a first heat exchanger in thermal contact with the permeate withdrawal line of the first module, a second heat exchanger in thermal contact with at least one permeate backfeed line of the subsequent modules, and a coolant supply line in fluid communication with at least one of the first and second heat exchangers. These internal heat exchange (cooling) configurations make it easier to control of the operating temperature of the process, which, in turn, facilitates control over yields, capacity and/or product purity in a consistent and reproducible manner.

According to other various embodiments, the filtration system has a permeate side exit for each of the subsequent modules after the first module, which has a respective permeate backfeed line entering the inlet side of a directly preceding module within the system, and a respective tap line for diverting permeate flow from the backfeed line for withdrawing permeate from the system. In this embodiment, permeate tap lines on one or more of the subsequent modules can be used to draw or pull off controlled amounts of permeate at one or more of the downstream stages. The drawn off permeate(s) can be used in blending operations with other permeate streams of the system, such as permeate drawn from the first module and or other subsequent stages, such that the overall purity of finished products can be adjusted to desired values using the TFF system.

According to various embodiments, the filtration process can provide an ability to run each stage independently as regards a driving force for separation (TMP for the TFF system) and to control filtration rate and purity. Unless indicated otherwise, the terms "stage" and "module" are used interchangeably herein.

Processes and/or methods using the filtration system, and products of the processes, are also provided. In the methods and systems described herein, it should be understood that the inlet side refers to the retentate side of the filtration module and the outlet side refers to the permeate side of the filtration module.

According to various embodiments, an industrial scale, cost effective process is provided that can recover proteins, for example enzymes. The process can utilize microfiltration to separate proteins from a fermentation broth thereby reducing process steps and process raw material addition. The process can further result in a high yield of protein products having high purity, low odor, low color, and/or increased efficacy as compared to protein products recovered using conventional industrial scale processes.

The systems and methods described herein can be used for separating and/or purifying products of interest. In various embodiments, the products can be produced with improved yields and have improved qualities. Improved yields and improved product qualities, however, aren't necessarily a requirement of the systems/apparatuses/processes described herein.

### BRIEF DESCRIPTION OF THE DRAWINGS

The skilled artisan will understand that the drawings described below, are for illustration purposes only. The drawings are not intended to limit the scope of the application teachings in any way.

Fig. 1 illustrates the spectrum of filtration processes.

Fig. 2 is a simplified drawing of a traditional microfiltration or TFF system.

Fig. 3 is a simplified drawing of an embodiment of the invention illustrating a modified TFF microfiltration apparatus or system.

Fig. 4 is a schematic of an embodiment of the invention.

Fig. 5 is a schematic of another embodiment of the invention.

Fig. 6 illustrates data obtained from an experiment investigating filtration parameters.

Fig. 7 illustrates data obtained from an experiment investigating filtration parameters, wherein the host organism and enzyme in the feed broth is *B. licheniformis* alpha-amylase.

Fig. 8 illustrates data obtained from an experiment investigating filtration parameters, wherein the host organism and enzyme in the feed broth is *T. reesei* cellulase.

Figs. 9A and 9B are tables providing exemplary operational examples with respect to the system of Figs. 4 and 5, illustrating measures to adjust (increase) one of the operational parameters of product yield, product purity, net permeation rate, and overall flux while maintaining the other three parameters substantially constant.

Fig. 10 is a schematic of the embodiment of Fig. 4 including an exemplified control system and process control components integrated therewith.

### DESCRIPTION OF VARIOUS EMBODIMENTS

It is to be understood that the following descriptions are exemplary and explanatory only. The accompanying drawings are incorporated in and constitute a part of this application and illustrate several exemplary embodiments with the description. Reference will now be made to various embodiments, examples of which are illustrated in the accompanying drawings.

Throughout the application, descriptions of various embodiments use "comprising" language, however, it will be understood by one of skill in the art, that in some specific instances, an embodiment can alternatively be described using the language "consisting essentially of" or "consisting of."

For purposes of better understanding the application and in no way limiting the scope of the teachings, it will be clear to one of skill in the art that the use of the singular includes the plural unless specifically stated otherwise. Therefore, the terms "a," "an" and "at least one" are used interchangeably in this application.

Unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. In some instances, "about" can be understood to mean a given value ±5%. Therefore, for example, about 100 ml, could mean 95-105 ml. At the very least, each numerical parameter should at least be construed in light of the number of reported significant digits and by applying ordinary rounding techniques.

According to various embodiments, methods are provided that refer to processes or actions involved in sample preparation or other procedures. It will be understood that in various embodiments a method or process can be performed in the order of processes as presented, however, in related embodiments the order can be altered as deemed appropriate by one of skill in the art in order to accomplish a desired result.

Biological broth should be understood to mean raw biological fluid produced by culture or fermentation of biological organisms, such as bacteria, fungi, mammalian or insect cells, or plant cells. The biological broth can contain a desired product, fermentation media and cells, or cell debris. Biological broths can also be obtained by extraction from biological samples such as plant matter or animal tissues or can mean the use of process intermediates, such as precipitates, crystals or extracts.

Cell separation should be understood to mean the process by which cells, cell debris, and/or particulates are removed to allow separation and recovery of desired compounds and to clarify a broth for further processing. Cell lysis procedures can precede cell separation.

Clarification should be understood to mean the removal of particulate matter from a solution.

Cell paste should be understood to mean material in the retentate portion of the filtration module; often it refers to the retentate that exits the last module.

Concentration should be understood to mean the removal of water from a broth, and can refer to the use of a membrane such as in microfiltration, ultrafiltration, nanofiltration or reversed osmosis processes, chromatography, precipitation, and crystallization. Concentration can also be accomplished by evaporation techniques.

Concentration Polarization should be understood to mean the accumulation of the retained molecules (gel layer) on the surface of a membrane and can be caused by a combination of factors: transmembrane pressure, crossflow velocity, sample viscosity, and solute concentration.

Concentration Ratio can be understood to mean the concentrate or retentate flow out of the module divided by the feed flow into the module.

Counter-current should be understood to mean backfeeding of permeate stream into the retentate stream at a location (stage) upstream of where the specific permeate was produced. As such, counter-current can refer to multistage backfeeding of permeate streams. A counter-current ultrafiltration process is described in: F. Lipnizki et. al., Desalination 144 (2002) 179-184.

It should be noted that the term counter-current as used herein is not to be confused with the term counter-current as is practiced in hemodialysis, which is described in S. Eloot et. al., Int. J. Artif. Organs (2004) 205-13.

It should be understood that counter-current flow is a multistage process where water, buffers, or salt solutions can enter the n^{th} stage, last stage, or other stage of a process opposite to the flow of the liquid containing a desired compound, but the counter-current flow can then be successively supplemented with liquid containing the desired compound as it moves to earlier stages.

Crossflow Velocity should be understood to mean velocity of the fluid flowing parallel to the membrane surface on the retentate side.

Diafiltration should be understood to mean the fractionation process by which smaller components are washed through the membrane, leaving the desired larger components in the retentate. It can be an efficient technique for removing or exchanging salts, buffers, removing detergents, low molecular weight materials, or changing the ionic or pH environment. The process can typically employ a microfiltration or ultrafiltration membrane that is employed to separate a product of interest from a mixture while maintaining the concentration of the larger component constant. Diafiltration can be accomplished with, for example, filtration permeate, water or a buffered salt solution.

Feed or Feedstream should be understood to mean the biological broth or raw material or raw solution at the beginning of a process containing a protein or other molecule of interest and which may also contain various contaminants including microorganisms, viruses and cell fragments.

The term fluids is used in a general sense, and unless indicated otherwise in a particular context, can encompass liquid materials containing dispersed and or solubilized species, pure liquids, or other flowable materials.

Flux or Overall Flux is the total volumetric rate of permeation divided by the total area of membrane in operation, usually expressed in liters per square meter per hour e.g. liters/m²/hour (lmh).

Fouling should be understood to mean obstruction of pores in a membrane by a gel layer, by a cell cake or cell paste, or by internal binding of molecules to the membrane pores.

Fractionation should be understood to mean the preferential separation of molecules based on a physical or chemical moiety.

Gel Layer or Boundary Layer should be understood to mean the microscopically thin layer of compounds that can form on the top of a membrane. It can affect retention of molecules by clogging, or fouling, the membrane surface and thereby reduce the flux.

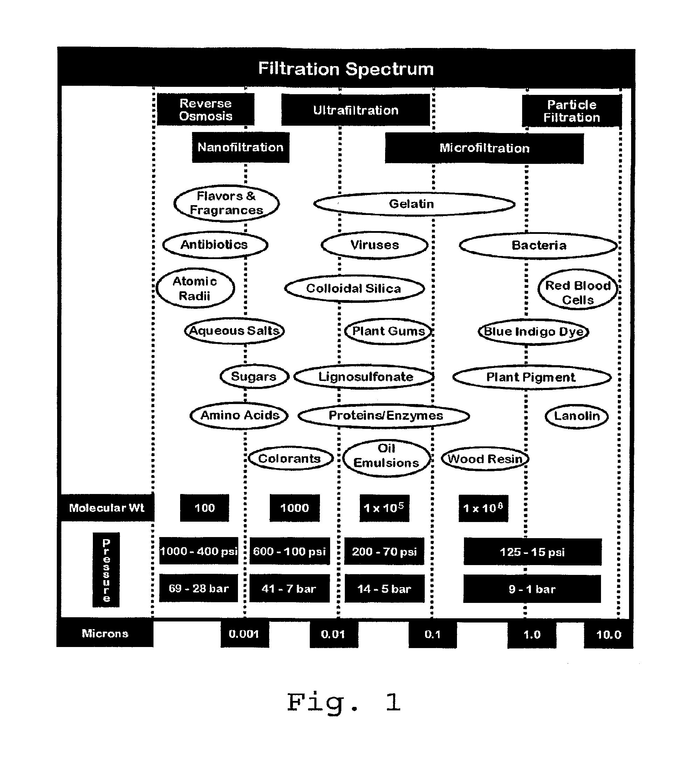

Microfiltration should be understood to mean a process that employs membranes to separate larger compounds from smaller compounds, for example, larger molecular weight from smaller molecular weight compounds. It can be used to concentrate mixtures and its efficiency is determined by factors such as the molecular weight cut off or pore size and type of the filter media, processing conditions and properties of the mixture being separated. The lower molecular weight compounds can be larger than the lower molecular compounds separated by ultrafiltration. Relative separation capabilities between ultrafiltration and microfiltration capabilities can be found described in Fig. 1. Of course, it will be noted that there is some overlap between the two filtration processes. The system and methods described herein, however can be applicable to all filtrations including, for example, RVDF, filter press and membrane systems as purification systems (for example, UF membrane). In the present invention microfiltration can be used to separate suspended particles in the range of about 0.05 to 10 microns, in the range of about 0.1 to 8 microns, and in the range of about 1 to about 5 microns from biological fluids, for example fermentation broth.

Molecular Weight Cut Off (MWCO) should be understood to mean the size (kilodaltons) designation for the ultrafiltration membranes. The MWCO is defined as the molecular weight of the globular protein that is 90% retained by the membrane

Net Permeation Rate is the flow rate of permeate leaving the system, typically expressed in liters per minute. The net permeation rate is a measure of the system throughput or capacity.

Passage or transmission should be understood to mean the movement of the protein/molecule through the membrane. In practice, passage is determined by calculating the ratio of permeate concentration to retentate concentration of the protein/molecule and is typically expressed as a percentage.

Permeation Rate is the flow rate, or the volume of permeate per unit time, flowing through a membrane and is typically expressed in liters per minute.

Product Yield or Yield is the total amount of product collected in the product stream, usually expressed as a percentage of the total amount in the feedstream. In various embodiments, one can obtain a high yield of a high purity product. In other embodiments, one can obtain a high yield of a product, however, the product may have a lower level of purity, for example, as in producing a crude extract from the retentate side of the filtration module.

Proteins, polypeptides or biologically derived polymers should be understood to mean molecules of biological or biochemical origin or in vitro processes. These are made of condensed building blocks of amino acids and include enzymes, structural proteins and cell derived polymers such as celluloses, starch, polyhydroxybutyric acid, and polylactate.

Product Purity or Purity is the degree of isolation of the product in the product stream. It can be understood to mean the amount of desired compound isolated compared to the sum amount of the other components in the stream and can be expressed as a weight percentage. Alternatively, it can be understood to mean the ratio of the concentration of the product relative to that of another selected component in the product stream and can be expressed as a number having the units of concentration divided by concentration. In various embodiments, purity is measured directly or indirectly instrumentally or manually, such as by determination of enzymatic activity (e.g., as determined colorimetrically); and or by product color determination by absorbance measurement, CIELAB formula, or US Pharmacopeia (USP) Monographs, and so forth to measure product color; and or by impurity level measurement (e.g., measurement of microbial impurities in fresh product or as part of shelf life studies); and or total protein content or other product component; and or organoleptically by odor, taste, texture, visual color, and so forth (e.g., in fresh product or as part of shelf life studies).

Rejection should be understood to mean the inability of the compounds to pass through the filter media because of, for example, the formation of a gel or boundary layer on a membrane surface; electrostatic charge interactions between the compound and a membrane surface; or the small pore size of the membrane.

Tangential Flow Filtration (TFF) should be understood to mean a process in which the fluid mixture containing the components to be separated by filtration is flowed at various velocities tangential to the plane of the membrane to minimize or limit gel layer formation or fouling. The feed material is pumped in a parallel direction to the membrane surface at a constant flow. In such filtrations a pressure drop can be created along the length of the membrane module. This filtration is suitably conducted as a batch process as well as a continuous-flow process. For example, the feed material may be passed repeatedly over the membrane while that fluid which passes through the filter is continually drawn off into a separate unit or the solution is passed once over the membrane and the fluid passing through the filter is continually processed downstream. Delta pressure or Delta P is the pressure drop along the flow path on the inlet side and can be defined as the difference between the feed and exit pressures on the inlet side, as measured in psi or bar.

Transmembrane Pressure (TMP) should be understood to mean the pressure differential driving force through the membrane to cause fluid and filterable solutes to flow through the filter. In tangential flow systems, highest TMPs are at the inlet (beginning of flow channel) and lowest at the outlet (end of the flow channel). TMP is calculated as an average pressure of the membrane module feed and exit on the retenate side, minus the pressure at the permeate side and can be expressed as pressure units such as psi or bar. Because flux can often vary with changes to TMP, flux is sometimes reported normalized to TMP, for example, as liters per square meter per hour per bar (1/m²/h/bar).

Retentate should be understood to mean the portion of the sample that does not pass through the membrane, also known as the concentrate. Retentate can be re-circulated during the TFF and is the mixture which stays on the feed side of the membrane.

Ultrafiltration should be understood to mean a process that employs membranes to separate large molecular weight compounds from low molecular weight compounds. It is used to concentrate solution and its efficiency is determined by the molecular weight cut off of the membrane. Relative separation capabilities between ultrafiltration and microfiltration capabilities can be found described in Fig. 1. Of course, it will be noted that there is some overlap between the two filtration processes. Ultrafiltration can be used to concentrate suspended solids and solutes of molecular weight greater than 1,000 daltons, and a size greater than about 0.005 microns and up to about 0.1 microns.

According to various embodiments, all of the methods, apparatuses, and systems described herein relate to aqueous solutions of proteins, polypeptides and biologically produced polymers and small molecule compounds, which can be in a mixture of viruses or cells (bacterial, fungal, amphibian, reptilian, avian, mammalian, insect, plants or chimeras), cell debris, residual media components, undesired biopolymers produced by the host cells, and contaminants introduced to the system during broth treatment which can occur in preparation for microfiltration. The methods, apparatuses and systems can also be used for the processing of feedstreams that are produced during the recovery of desired molecules, such as precipitates, solvents of aqueous extracts, and crystal slurries. In various embodiments, a filtration system can comprise a filtration apparatus, however, in some embodiments reference to a filtration system can be used interchangeably with reference to a filtration apparatus or filtration machine.

According to various embodiments a filtration system is provided that can comprise: a system inlet that can be configured to receive a feedstream; a first filtration module comprising a first inlet side and a first outlet side, where the first inlet side can be in fluid communication with the system inlet; a second filtration module comprising a second inlet side and a second outlet side, where the second inlet side can be in fluid communication with the first inlet side; and a first interruptable bypass that can be configured to define an interruptable fluid communication between the second outlet and the first inlet; and a system outlet that can be in fluid communication with each of the first outlet side and the second outlet side. In various embodiments, the system outlet can be in interruptable fluid communication with each of the first outlet side and the second outlet side. In various embodiments, the first filtration module and the second filtration module can comprise a tangential flow filtration module.

According to various embodiments, the system can comprise a heat exchanger in thermal contact with the first interruptable bypass and/or a coolant supply line in fluid communication with at least one of the first inlet side and the second inlet side or can be in fluid communication with each of the first inlet side and the second inlet side. In various other embodiments the system can comprise a temperature controlled device that can either heat or cool.

According to various embodiments, the system can comprise: a third filtration module comprising a third inlet side and a third outlet side, where the third inlet side can be in interruptable fluid communication with the second inlet side, and where the third outlet side can be in fluid communication with the system outlet side; and a second interruptible bypass can be configured to provide an interruptable fluid communication between the third outlet side and at least one of the first inlet side and the second inlet side.

According to various embodiments, the system can comprise a first heat exchanger that can be in thermal contact with the first interruptable bypass, and a second heat exchanger that can be in thermal contact with the second interruptable bypass.

According to various embodiments, the system can comprise a coolant supply line that can be in fluid communication with at least one of the first and second heat exchangers or can be in fluid communication with each of the first inlet side, the second inlet side, the first heat exchanger and the second heat exchanger. The coolant supply line can comprise a coolant bleed-off segment for diverting already circulated coolant from the system.

According to various embodiments, the system can comprise a feedstream, and the system inlet can be in fluid communication with the feedstream. The feedstream can comprise an enzyme broth or any fluid that may need to be filtered, purified or processed.

According to various embodiments, a filtration system is provided that can comprise: a system inlet configured to receive a feed stream; a first filtration module comprising a first inlet side and a first outlet side, where the first inlet side can be in fluid communication with the system inlet; a second filtration module comprising a second inlet side and a second outlet side, where the second inlet side can be in fluid communication with the first inlet side; a first interruptable bypass that can be configured to define an interruptable fluid communication between the second outlet side and the first inlet side; and a system outlet that can be in fluid communication with each of the first inlet side and the second inlet side. In various embodiments, the system outlet can be in interruptable fluid communication with each of the first inlet side and the second inlet side. In various embodiments, the first filtration module and the second filtration module can comprise a tangential flow filtration module.

According to various embodiments, the system can comprise a heat exchanger in thermal contact with the first interruptable bypass.

According to various embodiments, the system can comprise a coolant supply line in fluid communication with at least one of the first inlet side and the second inlet side or can be in fluid communication with each of the first inlet side and the second inlet side.

According to various embodiments, the system can comprise a first heat exchanger that can be in thermal contact with the first interruptable bypass; and a second heat exchanger that can be in thermal contact with the second interruptable bypass. In various embodiments, a heat exchanger can be in thermal contact with one or more interruptable bypasses.

According to various embodiments, the system can comprise a coolant supply line that can be in fluid communication with at least one of the first and second heat exchangers or can be in fluid communication with each of the first inlet side, the second inlet side, the first heat exchanger and the second heat exchanger. In various embodiments, the heat exchanger can be in thermal contact with one or more of the inlet sides or other portions of the system.

According to various embodiments, the system can comprise a feedstream, and the system inlet can be in fluid communication with the feedstream. The feedstream can comprise an enzyme broth or any fluid that may need to be filtered or processed.

According to various embodiments, a filtration system is provided that can comprise: a reservoir that can be in fluid communication with a fluid processing pathway; at least a first flow filtration module and a second flow filtration module, where the second flow filtration module can comprise a permeate outlet and the first flow filtration module can comprise a permeate backfeed inlet; a plurality of pumps and valves for pumping and regulating a flow of a fluid sample through the fluid processing pathway; and a plurality of conduits in fluid communication with the first and second flow filtration modules and the reservoir that can at least partially define the fluid processing pathway through which a fluid sample is flowed, where the fluid processing pathway can be configured to recycle a permeate through at least the first and second flow filtration modules, where the permeate outlet of the second flow filtration module can be in communication with the permeate backfeed inlet of the first filtration module, and where permeate from at least one of the first flow filtration module and the second flow filtration module can exit the system. In various embodiments, the fluid communication between the first flow filtration module and the second flow filtration module further can comprise a bypass for optional removal of the permeate from the second flow filtration module prior to backfeeding into the first flow filtration module. In various embodiments, the system can comprise at least one additional flow filtration module. The system can further comprise an additional vessel and pumps that a permeate may go through before reentering a filtration module.

According to various embodiments, each of the first flow filtration module and the second flow filtration module can comprise a tangential flow filtration module.

According to various embodiments, the system can comprise conduits defining a pathway for a fluid wash stream, separate and distinct from the fluid processing pathway, each of the conduits are in fluid communication with the reservoir and each of the first flow filtration module and the second flow filtration module.

According to various embodiments, the system can comprise a cooling system, and the cooling system can be in thermal contact with the fluid processing pathway. In various embodiments, the cooling system can be defined by conduits defining a cooling stream pathway separate and distinct from the fluid processing pathway, the conduits can be in fluid communication with each of the first flow filtration module and the second flow filtration module. The cooling stream within the cooling stream pathway can comprise a liquid, for example, diafiltering liquid.

According to various embodiments, each of the first flow filtration module and the second flow filtration module can comprise a membrane adapted to separate a species of interest from a mixture while a normalized flux is maintained at about 0.1 to about 200 L/m²/hr/bar, and wherein the flow filtration module is maintained under TMP of 0.1 bar to 60 bar. The term "bar" is defined as a unit of pressure corresponding to 10⁵ Pa. Conventional pressures can be considered to be in the range of about 0.1 to about 1.5 bar, however, this range can vary depending on for example, a protein being filtered or a filtration medium being used. High pressures can be considered to begin above about 1.5 to about 2.0 bar. The apparatus and methods described herein can operate at conventional and/or high pressures. In various embodiments the apparatuses and systems can operate from about 0.1 bar to about 60 bar, from about 0.1 to about 50 bar, from about 0.1 bar to about 40 bar, from about 0.1 bar to about 30 bar, from about 0.1 bar to about 20 bar, from about 0.1 bar to about 10 bar, from about 0.1 bar to about 5.0 bar, from about 0.5 bar to about 60 bar, from about 1.0 bar to about 60 bar, from about 1.5 bar to about 60 bar, from about 2.0 bar to about 60 bar, from about 2.5 bar to about 60 bar, from about 3.0 bar to about 60 bar, from about 3.5 bar to about 60 bar, from about 4.0 bar to about 60 bar, from about 4.5 bar to about 60 bar, from about 5.0 to about 60 bar, from about 1.0 bar to about 10 bar, from about 1.0 bar to about 5 bar, from about 5 bar to about 10 bar, from about 10 bar to about 30 bar, from about 30 bar to about 60 bar, or above 60 bar.

According to various embodiments, the filtration system can comprise a plurality of sensors for acquiring data about a fluid sample as it flows through the fluid process pathway. In various embodiments, the filtration system can comprise an electronic data processing network capable of at least receiving, transmitting, processing, and recording data associated with the operation of said pumps, valves, and sensors and the recorded data collected during a flow filtration process can be sufficiently comprehensive to control the flow filtration process.

According to various embodiments a filtration system is provided comprising: a reservoir; a plurality of filtration modules, each filtration module comprising a sample chamber; a plurality of conduits together with the reservoir and the plurality of filtration modules defining a fluid process stream through which a fluid sample is conducted, where the fluid process stream is separate and distinct from a cooling stream and a cleaning stream; the conduits together with the plurality of filtration modules and the reservoir defining the fluid processing stream; a fluid processing stream for recycling a permeate through at least two of the plurality of filtration modules; a plurality of pumps and valves for pumping and regulating the flow of the fluid sample through the fluid process stream; a plurality of conduits together with a cooling source and the plurality of filtration modules defining a cooling stream through which a cooling fluid is conducted, where the cooling stream is separate and distinct from the fluid process stream and the cleaning stream; a plurality of pumps and valves for pumping and regulating the flow of the cooling stream from a cooling source into the chamber of at least one of the plurality of filtration modules; a plurality of conduits together with the reservoir and at least one of the plurality of filtration modules defining a wash stream through which a cleaning fluid is conducted, where the cleaning stream is separate and distinct from the cooling stream and the fluid processing stream; and a plurality of pumps and valves for pumping and regulating the flow of the cleaning stream from the reservoir into the chamber of at least one of the plurality of filtration modules.

According to various embodiments, the cooling stream can comprise diafiltration fluid.

According to various embodiments, the fluid process stream can comprise a permeate outlet of a second flow filtration module in fluid communication with a permeate backfeed inlet of a first filtration module, and the system is adapted to collect permeate from the second or both the first and second filtration modules.

According to various embodiments, the fluid process stream between the first and second filtration module can comprise a bypass for optional removal of the permeate from the second flow filtration module prior to entry of the first filtration module.

According to various embodiments, the system can comprise a tangential flow filtration system and the filtration modules can be tangential flow filtration modules.

According to various embodiments, at least one of the plurality of flow filtration modules can comprise a membrane adapted to separate a species of interest from a mixture while a normalized flux is maintained at about 0.1 to about 200 L/m²/hr/bar, and the flow filtration module is maintained under TMPs from about 0.1 to about 60 bar.

In various embodiments, a filtration module can be controlled by a controller. The controller can play a role in regulating various parameters of the filtration process, for example purity, yield, TMP, CF, net permeation rate, or flux. The system can also comprise valves that assist in system regulation. An appropriate control scheme can be determined based upon the needs for filtering or purifying compounds of interest.

According to various embodiments, the filtration system can comprise a plurality of sensors for acquiring data about the fluid sample as it flows through said fluid process stream. In various embodiments, the system can comprise an electronic data processing network capable of at least receiving, transmitting, processing, and recording data associated with the operation of said pumps, valves, and sensors, detectors and the recorded data collected during said tangential flow filtration process can be sufficiently comprehensive to determine control of the tangential flow filtration process.

According to various embodiments, detectors can comprise detectors that measure flow rate, pressure, concentration, pH, conductivity, temperature, turbidity, ultraviolet absorbance, fluorescence, refractive index, osmolarity, dried solids, near infrared light, or Fourier transform infrared light. Such detectors can be used for monitoring and controlling the progress and safety of filtration procedures.

According to various embodiments, the filtration system can comprise a second cooling stream defined by a heat exchanger in fluid communication with a cooling source and at least one of the plurality of filtration modules, wherein the second cooling stream can be adapted to optionally prevent the cooling stream from being in communication with at least one of the plurality of filtration modules, but still permit flow of the cooling stream between the heat exchanger and cooling source.

According to various embodiments, a filtration system is provided that can comprise: a system inlet configured to receive a feedstream; a first filtration module comprising a first inlet side and a first outlet side, where the first inlet side can be in fluid communication with the system inlet; a second filtration module comprising a second inlet side and a second outlet side, where the second inlet side can be in fluid communication with the first inlet side; a coolant supply line that can be in fluid communication with each of the first inlet side and the second inlet side, where the coolant supply line is not in thermal communication with a heat exchanger; a system outlet that can be in fluid communication with each of the first outlet side and the second outlet side; and an additional system outlet that can be in fluid communication with each of the first inlet side and the second inlet side.

According to various embodiments, the system can comprise a filtration module that can be a Tangential Flow Filtration (TFF) module and the system or apparatus can be a TFF microfiltration apparatus or TFF filtration system. The apparatus can be designed or adapted to allow the operation of stages or individual steps under particular control setpoints for diafiltration in order to obtain a high level of purification control of products and byproducts.

Purification of a product can comprise about 10% to about 25% purity, about 25% to about 50% purity, about 50% to about 75% purity, about 75% to about 90% purity, about 90% to about 95% purity, about 95% to about 97% purity, or about 97% to about 99% purity, of the product of interest.

Purified or substantially purified can refer to proteins, nucleic acids or other compounds that are removed from their natural environment, or isolated or separated from a mixture, and are free from other components with which they are naturally associated on a percentage basis. For example, a 60% pure enzyme preparation can comprise 60% of the enzyme of interest and 40% other components, for example, other proteins or cell debris.

According to various embodiments, the system can comprise a TFF microfiltration apparatus designed or adapted to allow operating each of the stages or individual steps of the TFF systems under particular control setpoints for permeate recirculation streams to obtain a high level of purification control of products and byproducts.

According to various embodiments, the system can comprise a TFF microfiltration apparatus that is designed or adapted to allow operation of each of the stages or individual steps of the TFF systems under particular control setpoints for concentration ratio to obtain a high level of control over purification of products and byproducts.

According to various embodiments, the system can comprise a TFF apparatus that can be operated from two to ten stages, or greater than ten stages of successive permeate backfeed.

According to various embodiments, the TFF apparatus can be operated with membrane elements from one element to 25 elements, and as many as 500 elements, within a filter module, arranged consecutively or in parallel within the module.

According to various embodiments, the TFF apparatus can be operated with but not restricted to polymeric, ceramic, or stainless steel type material regardless of configuration, for example, spiral, tubular, hollow fiber, flat-sheet, etc.

Examples of various membrane materials that can be used in the membranes of the system or process can comprise polyvinylidene difluoride (PVDF), polysulfone, polyethersulfone, polyarylsulfone, regenerated cellulose, polyamide, polypropylene, polyethylene, polytetrafluoroethylene (PTFE), cellulose acetate, polyacrylonitrile, vinyl copolymer, polyamides, polycarbonate, or blends thereof or the like.

According to various embodiments, the system can comprise a TFF microfiltration apparatus that is designed or adapted such that each of the stages or steps are amenable to automation, and the automation can lead to a self-controlled system, however, in other embodiments the automation may not lead to a self-controlled system.

According to various embodiments, the TFF apparatus can be used in accordance with methods described herein. The TFF apparatus can have the ability to separate at high recovery yields molecules, compounds, and or particles both at low and high operating pressures that could not be performed with traditional apparatuses, for example, rotary drum vacuum filter (RDVF), filter press, or dead-end membrane filter. High recovery yields refer to yields of about 80% and above. In other embodiments, yields can be less than 80%, from about 60% to about 80%, from about 40% to about 60% or from about 20% to about 40%. As noted above, in various embodiments a preparation can have both a high yield and high purity; or high yield and low purity.

According to various embodiments, the TFF apparatus can purify product streams of undesirable product properties including but not restricted to odors, colors, or contaminants. Other properties of interest can also be removed or decreased or increased in the purified product.

According to various embodiments, a TFF system can comprise a mechanical apparatus to make differentiable product at equal or reduced manufacturing costs compared to conventional systems. In other embodiments, a TFF apparatus can reduce several chemical steps, processes, and/or procedures relative to conventional technologies and also produce superior (differentiable) product. For example, compared to a conventional process or product, an embodiment of the machine/apparatus/system of the invention has assisted in the removal of 3 processing steps, reduced exposure to equipment and employees, and improved efficiency by ≥ 7%. Efficiency can be defined by a combination of capacity, yield, cost, and/or flux improvements. In various embodiments, purity of product is also generally improved by ≥ 4%, particularly ≥ 10%, and more particularly ≥ 50% (as defined by an increase in the active to total protein ratio) relative to a permeate product of a similar enzyme source obtained by rotary drum vacuum filtration, wherein the percentage improvement in activity within these ranges can vary depending on the particular enzyme. In other various embodiments, TFF products of the present invention have activity/nitrogen improved by ≥ 3%, particularly ≥10%, and more particularly ≥ 50%, relative to a permeate product of a similar enzyme source obtained by rotary drum vacuum filtration. In other various embodiments, TFF products of the present invention have activity/carbon improved by ≥ 5%, particularly ≥10%, and more particularly ≥ 50%, relative to a permeate product of a similar enzyme source obtained by rotary drum vacuum filtration. In other various embodiments, a permeate product of microfiltration of an enzyme source is provided having at least a 40% decrease in color, wherein color is based on absorbance values measured at 470 and or 520nm using a spectrophotometer, relative to a permeate product of a similar enzyme source obtained by rotary drum vacuum filtration.

According to various embodiments, a TFF system can comprise an apparatus which produces differentiable product quality for at least one of the permeate product and retentate product. The soluble components or solutes in the permeate can have very low or no odor and higher purity (for example, a higher extent of isolation of a desired component) than materials produced in other systems or by other methods. The insoluble components in the retentate can have less odor and improved color. In various embodiments, formulations made from the recovered permeate can be distinguished from the same compounds made using conventional microfiltration processes as relates to the above properties.

According to various embodiments, a TFF system can comprise an apparatus that increases the sustainability of a filtration or purification process by reducing, for example, raw materials usage, power consumption, and/or recyclable waste stream (over conventional purification methods).

Various parameters can affect and control yield, purity, flux and net permeation rate of the systems and processes described herein.

Six exemplary TFF operating parameters and control relationships are as follows: 1) Surface area or number of modules can affect net permeation rate; 2) Diafiltration flow rate can affect purity, net permeation rate, yield, and flux; 3) Transmembrane Pressure can affect flux, net permeation rate, purity, and yield; 4) Crossflow Velocity can affect flux, net permeation rate, purity and yield; 5) Module type has specific physical characteristics which can satisfy flux, net permeation rate, yield and purity requirements of the separation to be performed; and 6) Diafiltration location can affect flux, net permeation rate, yield and purity based on injection point into the system.

The impact of these six parameters on the performance of a TFF system can vary depending on the specific properties of and interactions among the feed material, diafiltering solution, membrane element, and product compound.

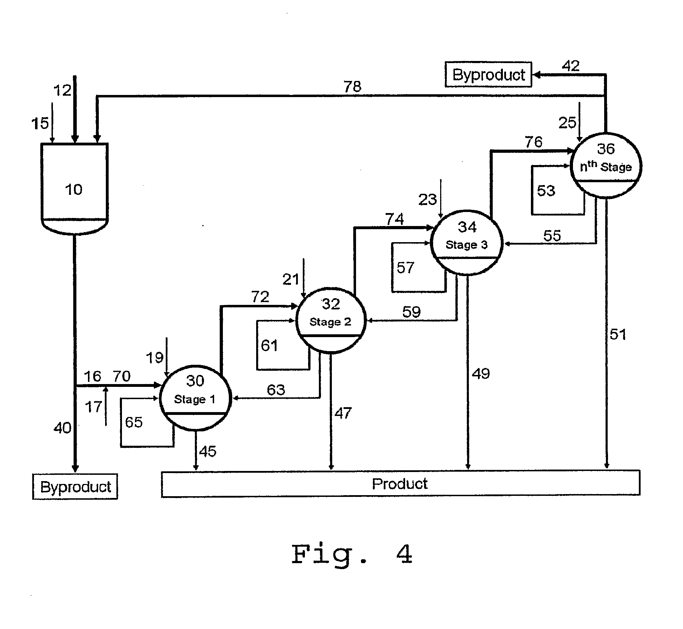

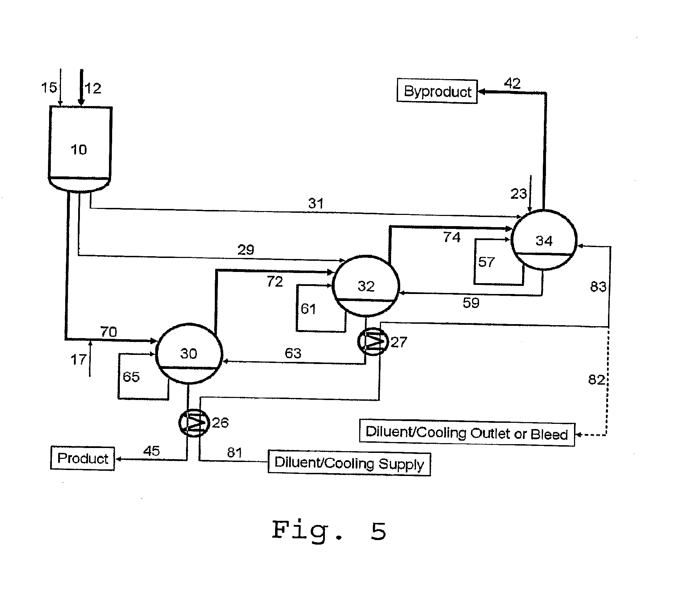

In addition to the above operating parameters, various embodiments can have three flows for the permeate from each module (Figs. 4 and 5). The combined operation of the three permeate streams can help to control flux, net permeation rate, yield and purity. Conventional TFF systems would not have all three permeate streams, nor the ability to control them independently. It is these three permeate streams and control of the above six operating parameters that can provide more specific and independent control of process outcomes, including product quality.

According to various embodiments, a TFF system can comprise a mechanical apparatus that enables a safer work environment because it can be fully enclosed and can reduce or eliminate employee exposure to hazardous materials (over conventional purification methods).

The apparatuses, machine and systems described herein can be used in various methods or processes. The processes are not limited to microfiltration processes, however, microfiltration processes are frequently referred to in exemplary embodiments.

According to various embodiments a microfiltration process can be combined with an ultrafiltration process which can include single stage or multistage counter-current processes or other filtration processes. In various embodiments, a microfiltration method can be combined with other recovery processes, for example, crystallization, centrifugation, evaporation, and column chromatography.

According to various embodiments, a filtration process is provided that can comprise: flowing a feed liquid through a feed conduit into an inlet side of a first filtration module; flowing a diafiltration solution into the inlet side of the first filtration module through a first diafiltration conduit separate from the feed conduit; separating the feed liquid and diafiltration solution into a first retentate and a first permeate, in the first filtration module; flowing the first retentate into an inlet side of a second filtration module through a retentate conduit; flowing a second diafiltration solution into the inlet side of the second filtration module through a second diafiltration conduit without flowing the second diafiltration solution through any other filtration module; separating the first retentate and the second diafiltration solution into a second retentate and a second permeate, in the second filtration module; and collecting at least one of the first permeate and the second permeate. In various embodiments, a fluid being filtered or purified can go through a separate vessel and pump prior to reentering a filtration module or the system.

According to various embodiments, the process can repeatedly filter a liquid through multiple additional filtration modules further comprising: flowing a retentate from one or more of the first filtration module, the second filtration module, or one of the additional filtration modules, into another filtration module; flowing a diafiltration solution from a conduit separate from the feed conduit into one or more of the additional filtration modules; backfeeding or recirculating one or more permeate or retentate as required to obtain a desired level of purity of a component in the permeate or the retentate; and collecting the permeate or retentate having the component of interest. In various embodiments, the compounds, or components of interest can be a protein, a polypeptide, a nucleic acid, a glycoprotein, another biopolymer, or a small molecule compound.

In various embodiments, the compounds can comprise therapeutic proteins such as antibodies, enzymatically active protein therapeutics (enzymes), and hormones. They can also comprise, for example, structural proteins such as collagen, elastin and related molecules. Hormones can include, but are not limited to, a follicle-stimulating hormone, luteinizing hormone, corticotropin-releasing factor, somatostatin, gonadotropin hormone, vasopressin, oxytocin, erythropoietin, insulin and the like. Therapeutic proteins can include, but are not limited to, growth factor, which is a protein that binds to receptors on the cell surface with the primary result of activating cellular proliferation and/or differentiation, platelet-derived growth factor, epidermal growth factor, nerve growth factor, fibroblast growth factor, insulin-like growth factors, transforming growth factors and the like.

According to various embodiments enzymes can be produced by an industrial scale process. Any enzyme may be used, and a nonlimiting list of enzymes include phytases, xylanases, β-glucanases, phosphatases, proteases, amylases (alpha or beta), glucoamylases, cellulases, phytases, lipases, cutinases, oxidases, transferases, reductases, hemicellulases, mannanases, esterases, isomerases, pectinases, lactases, peroxidases, laccases, other redox enzymes and mixtures thereof.

In some embodiments the enzyme recovered is a hydrolase, which includes, but is not limited to, proteases (bacterial, fungal, acid, neutral or alkaline), amylases (alpha or beta), lipases, cellulases, and mixtures thereof, for example, enzymes sold under the trade names Purafect®, Purastar®, Properase®, Puradax®, Clarase®, Multifect®, Maxacal®, Maxapem®, and Maxamyl® by Genencor International (USP 4,760,025 and WO 91/06637); Alcalase®, Savinase®, Primase®, Durazyme®, Duramyl®, Clazinase®, and Termamyl® sold by Novo Industries A/S (Denmark).

Cellulases are enzymes that hydrolyze the β-D-glucosidic linkages in celluloses. Cellulolytic enzymes have been traditionally divided into three major classes: endoglucanases, exoglucanases or cellobiohydrolases and β-glucosidases (J. Knowles et al., TIBTECH (1987) 5:255-261). An example of a cellullase is Multifect® BGL, available from Genencor International. Cellulases can be made from species such as *Aspergillus, Trichoderma, Pereicillium, Humicola, Bacillus, Cellulomonas, Thermomonospore, Clostridium,* and *Hypocrea.* Numerous cellulases have been described in the scientific literature, examples of which include: from *Trichoderma reesei,* S. Shoemaker et al., Bio/Technology (1983) 1:691-696, which discloses CBHI; T. Teeri et al., Gene (1987) 51:43-52, which discloses CBHII; M. Penttila et al., Gene (1986) 45:253-263, which discloses EGI; M. Saloheimo et al., Gene (1988) 63:11-22, which discloses EGII; M. Okada et al., Appl Environ Microbiol (1988) 64:555-563, which discloses EGIII; M. Saloheimo et al., Eur J Biochem (1997) 249:584-591, which discloses EGIV; and A. Saloheimo et al., Molecular Microbiology (1994) 13:219-228, which discloses EGV. Exo-cellobiohydrolases and endoglucanases from species other than *Trichoderma* have also been described e.g., *Ooi et al.,* 1990, which discloses the cDNA sequence coding for endoglucanase F1-CMC produced by *Aspergillus aculeatus;* T. Kawaguchi *et al.,* 1996, which discloses the cloning and sequencing of the cDNA encoding beta-glucosidase 1 from *Aspergillus aculeatus*; Sakamoto *et al.,* 1995, which discloses the cDNA sequence encoding the endoglucanase CMCase-1 from *Aspergillus kawachii* IFO 4308; and Saarilahti *et al.,* 1990 which discloses an endoglucanase from *Erwinia carotovara.*

Proteases, include, but are not limited to serine, metallo, thiol or acid protease. In some embodiments, the protease will be a serine protease (e.g. subtilisin). Serine proteases are well known in the art and reference is made to Markland et al., Honne-Seyler's Z Physiol. Chem (1983) 364:1537 - 1540; J. Drenth et al. Eur J Biochem (1972) 26:177 - 181; U.S. Pat. Nos. 4,760,025 (RE 34,606), 5,182,204 and 6,312,936 and EP 0 323,299. Means for measuring proteolytic activity are disclosed in K.M. Kalisz, "Microbial Proteinases" Advances in Biochemical Engineering and Biotechnology, A. Fiecht Ed. 1988.

Xylanases include, but are not limited to, xylanases from *Trichoderma reesei* and a variant xylanase from *T. reesei,* both available from Danisco A/S, Denmark and/or Genencor International, Inc., Palo Alto, California as well as other xylanases from *Aspergillus niger, Aspergillus kawachii, Aspergillus tubigensis, Bacillus circulans, Bacillus pumilus, Bacillus subtilis, Neocallimastix patriciarum, Streptomyces lividans, Streptomyces thermoviolaceus, Thermomonospora fusca, Trichoderma harzianum, Trichoderma reesei, Trichoderma viride.*

Examples of phytases are Finase L®, a phytase from Aspergillus sp., available from AB Enzymes, Darmstadt, Germany; Phyzyme™ XP, a phytase from *E. coli,* available from Danisco, Copenhagen, Denmark, and other phytases from, for example, the following species: *Trichoderma, Penicillium, Fusarium, Buttiauxella, Citrobacter, Enterobacter, Penicillium, Humicola, Bacillus,* and *Peniophora.*

Amylases may be, for example, from species such as *Aspergillus, Trichoderma, Penicillium, Bacillus,* for instance, *B. subtilis, B. stearothermophilus, B. lentus, B. licheniformis, B. coagulans,* and *B. amyloliquefaciens.* Suitable fungal amylases are derived from *Aspergillus,* such as *A. oryzae* and *A. niger.* Proteases may be from *Bacillus amyloliquefaciens, Bacillus lentus, Bacillus subtilis, Bacillus licheniformis,* and *Aspergillus* and *Trichoderma* species.

The above enzyme lists are examples only and are not meant to be exclusive. For example, other enzyme-producing host organisms may include *Mucor sp, Kluyveromyces sp, Yarrowia sp, Acremonium sp, Neurospora sp, Myceliophthora sp,* and *Thielavia sp.* Any enzyme may be used in the present invention, including wild type, recombinant and variant enzymes of bacterial, fungal, plant and animal sources, and acid, neutral or alkaline pH-active enzymes.

According to various embodiments, this process can also be used for the purification of biologically produced polymers, such as polylactic acid, polyhydroxybutiric acid and similar compounds. In no way, however, is the method or apparatus intended to be limited to preparation or processing of the above polymers.

According to various embodiments, this process can also be used for the purification of biologically produced small molecule compounds, such as vitamins (e.g. ascorbic acid), ethanol, propanediol, amino acids, organic dyes (e.g. indigo dye), nutraceuticals (e.g. betaine and camitine), flavors (e.g. butyl butyrate), fragrances (e.g. terpenes), organic acids (e.g. oxalic, citric, and succinic acids), antibiotics (e.g. erythromycin), pharmaceuticals (e.g. statins and taxol), antioxidants (e.g. carotenoids), sterols, and fatty acids. In no way, however, is the method or apparatus intended to be limited to preparation or processing of the above small molecule compounds.

The desired purity of the component or compound of interest in the permeate, retentate or cell paste can be from about 25% to about 100%. In various embodiment, the purity of the component of interest can be from about 5% to about 25% pure, about 25% to about 50% pure, from about 50% to about 75% pure, from about 75% to about 90% pure, from about 90% to about 95% pure, from about 95% to about 97% pure or from about 97% to about 99% pure.

According to various embodiments, the permeate from one of the filtration modules can be recirculated (backfed) into the retentate of the previous filtration module. In various embodiments, a liquid from a cooling source can be pumped directly into the retentate in at least two filtration modules, without prior recirculation of the cooling liquid between multiple filtration modules.

According to various embodiments, the process can comprise running a multi-stage counter-current flow, and filtering a sample through from two to about 20 filtration modules.

According to various embodiments, the process can comprise flowing a permeate or retentate through one or more additional filtration modules. In various embodiments, the first filtration module can comprise a membrane and the process can comprise maintaining a transmembrane pressure of from about 0.1 bar to about 60 bar, from about 2 bar to about 10 bar or at transmembrane pressure greater than about 60 bar. In various embodiments, the transmembrane pressure can be measured in the filtration module

According to various embodiments, the process can comprise flowing a liquid through the first filtration module. The liquid can comprise at least one of water, salt, buffer, and detergent solution. In various embodiments, the process can comprise counter-current flow of the liquid through at least one filtration module.

According to various embodiments, the counter-current liquid can comprises a detergent solution, and the detergent solution can comprise from about 1% to about 25% sodium chloride, and at least one of sodium sulfate, sodium phosphate and a buffer.

According to various embodiments, at least one of the filtration or separating steps in the process can be carried out at a temperature of from about 1° C to about 100° C, from about 1° C to about 25° C, from 25° C to about 50° C, or from about 50° C to about 75° C, or from about 75° C to about 100° C. In various embodiments, at least one of the filtering or separating steps can be carried out at a pH of from about pH 1 to about pH 14, from about pH 2 to about pH 10, from about pH 3 to about pH 9, from about pH 4 to about pH 8, or pH 5-pH 14.

According to various embodiments, in the method, at least one of the first and second filtration modules can comprise at least one of a ceramic filter, a stainless steel filter, a hollow fiber filter, a tubular filter, a spiral filter, a flat sheet filter, or other filter configuration. In various embodiments, the filter can have a filter pore size of from about from about 0.02 micron to about 0.05 microns, 0.05 micron to about 10 microns, from about 0.05 micron to about 0.5 microns, from about 0.5 microns to about 1 microns, from about 1 micron to about 5 microns, from about 5 microns to about 10 microns, or from about 10 microns to about 100 microns.

According to various embodiments, the pore size between different stages of the process can be either the same or may differ from stage to stage.

Various tangential flow filtration module configurations are known to those of skill in the art. Several types are described in the patent literature, for example in, U.S. Pat. No. 6,054,051, U.S. Pat. No. 4,761,230 U.S. Pat. No. 5,096, U.S. Pat. No. 5,256,294, and U.S. Pat. No. 5,525,144, all of which are incorporated herein in their entirety by reference. Additionally, flow filtration modules are commercially available, for example, "Pellicon XL" and "Pellicon 2" TFF cartridges (available from Millipore Corporation of Bedford, Mass. 01730); and "Centramate", "Centrasette", "Maximate" and "Maximate-Ext" TFF cartridges (available from Pall Corporation of East Hills, N.Y. 11548).

According to various embodiments, the methods can comprise optimizing system parameters. The system parameters can comprise at least one of temperature, feedstream flow velocity, transmembrane pressure, feedstream concentration and diafiltration volume. Feedstream velocity can range from about 1cm/sec to about 500 cm/sec. Feedstream concentration can vary as deemed appropriate. Dialfiltration volume can range from about 1 time to about 100 times retentate volume.

According to various embodiments, the feed liquid for the process can be obtained from a production organism or production cells. The production organism can be a virus, bacteria or fungus. Production cells can comprise prokaryotic or eukaryotic cells. In various embodiments, the production cells can comprise bacterial cells, insect cells, mammalian cells, fungal cells, plant cells, or a cell line from the previously referred to cells. Cell lines can comprise cells from mammals, birds, amphibians or insects. The cells can be transformed or transfected with DNA or other nucleic acids of interest, such that the cells express a biopolymer of interest. Methods of cell transformation and/or transfection are well-known in the art and can be found for example in US Patent No. 7,005,291, which is incorporated herein in its entirety by reference.

In various embodiments, the feed liquid can be obtained from non-transformed or non-transfected cells or from other sources, for example, animal or plant tissue, such that the feed liquid obtained from the source can be flowed through a multistage-filtration apparatus. In various other embodiments feed liquid can be obtained from transgenic cell or organisms, for example, transgenic mammals. Results of the process can be independent from the starting or raw material entering the process as feed liquid. The process can be applied to broths obtained from the extraction of plant or animal matter and process intermediate, or final forms of products that can comprise crystal slurries, precipitates, permeates, retentates, cell paste or extracts.

According to various embodiments, a bacterial production organism can be from any bacterial species, such as *Bacillus, Streptomyces* or *Pseudomonas* species, for instance from *Bacillus subtilis, Bacillus clausii, Bacillus licheniformis, Bacillus alkalophilus, Escherichia coli, Pantoea citrea, Streptomyces lividans, Streptomyces rubiginosus* or *Pseudomonas alcaligenes.*

According to various embodiments, a retentate can comprise a cell paste and the process can comprise collecting the cell paste.

According to various embodiments, the microfiltration process can be operated at transmembrane pressures that enable the formation of a gel or boundary layer. Pressures can range from 0.1 to about 60 bar. The lower limit of the range can be determined by the choice of membrane system.

According to various embodiments, the process can be run in a continuous-flow mode, batch mode, counter-current mode or various combinations of modes as deemed appropriate. In a counter-current mode, permeate streams can be fed back into the inlet side of the various stages of the filtration modules.

In another embodiment, the process is operated in fully enclosed equipment, thus eliminating exposure risks and the need to engineer environmental controls.

In another embodiment, the process retentate, i.e. concentrated cells and cell debris can contain little or no environmentally detrimental compounds such as synthetic flocculants and .can thus be recycled or disposed of without special environmental disposal measures.

According to various embodiments, the process can produce a product with improved product qualities such as, but not limited to, lower odor, lower color, higher or lower concentration.

In various embodiments, the diafiltration solution can be used more efficiently because it can be used to extract compounds multiple times. After the first extraction , it can carry a few compounds, and then more and more until the diafiltration solution exits the system. By controlling where (for example, which stage) it exits the system, one can control the purity of the compounds on the retentate or the permeate side of the membrane.

According to various embodiments, under similar filtration conditions used in conventional processes, except for transmembrane pressure, a higher yield and purer product can be obtained using the processes described herein when compared to the yield and product purity obtained in the conventional processes using relatively lower transmembrane pressures.

The separation principles in low TMP microfiltration technology can be similar to high TMP microfiltration technology described herein, however low TMP can result in lower product quality. This can be due to the fact that low TMP processing, typically has to run at lower pressures such that the boundary layer filtering effect is less pronounced.

The TFF microfiltration process described herein can produce a higher quality product at lower production costs. Higher purity can be achieved through the formation of a hydrodynamic layer (gel or boundary layer) on the surface of the membrane. This layer can be formed due to the higher transmembrane pressure. The resulting lower transmission and higher compound rejection at higher pressures can be compensated for by the unique counter-current design such that it can provide an overall yield which results in an economically acceptable process The counter-current design described herein can increase yields because it can allow for the diafiltration solution to be used more effectively. This can allow one to achieve better separation of compounds and can result in greater recovery yield and purity when compared to conventional TFF.

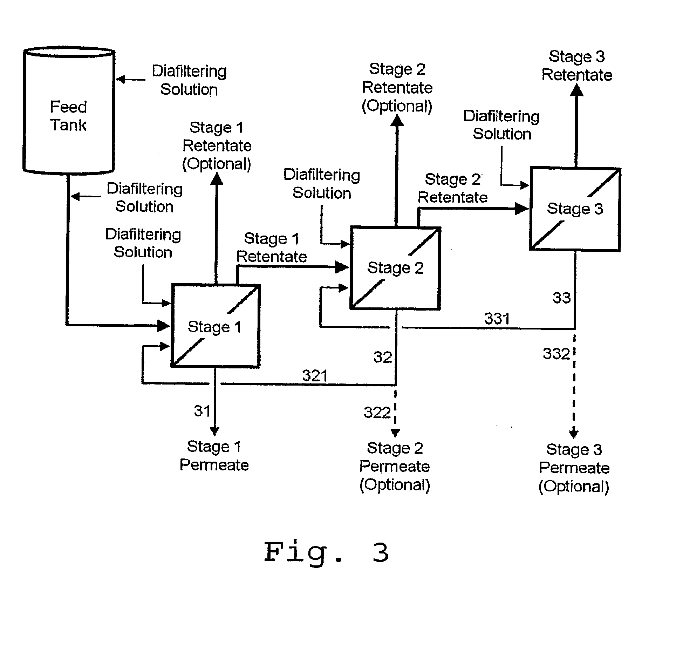

The processes described above can use a combination of counter-current and diafiltration, but is not solely restricted to membrane related processes, although it is most commonly used in this manner. Various separation technologies (rotary vacuum drum filter, filter press, membrane filter press, chromatographic separations, decanters, phase separations by density or polarization changes, etc.) can also be applied in place of a single stage of membranes. Fig. 3 illustrates different options for addition of a diafiltration solution as well as the removal of permeate from the various stages that are being used for separation of the molecule of interest.

In various embodiments, the invention provides a process for separating proteins, polypeptides and biologically produced polymers and small molecule compounds from biological broths using microfiltration apparatus system, and methods described herein can provide cost effective capital installation, cost effective operation, improved product quality, high product yield, elimination of most or all cell separation raw material consumption, minimized exposure of operators to potentially hazardous compounds, and recycling of liquid stream for useful applications.

The process described herein can also provide a higher level of product purity than traditional methods or systems.

According to various embodiments, the apparatuses, systems and methods described herein can use fluid obtained after lysis of production cells or other materials. Methods of lysing are well-known in the art and are described, for example, in US Patent Application Publication 200/0014245, which is incorporated herein in its entirety by reference.

TFF, as well as other filtration processes, can be used to separate different molecules (or particles) of different sizes. A broad spectrum of molecules or compounds can be separated using a variety of pressures and filter pore size cut-offs. Different TFF processes can be specific for different particle size ranges of desired molecules and/or particles that are intended to be separated or purified. As illustrated in Fig. 1, Microfiltration (MF), Ultrafiltration (UF), Nanofiltration (NF), and Reverse Osmosis (RO) can be used to separate or purify molecules (or particles) in the molecular weight or size range of 0.1-10 µm, 0.005-0.lum, 100-1000 daltons molecular weight, and 0-500 daltons molecular weight, respectively. These ranges can vary somewhat, as indicated at the bottom of Fig. 1.

A characteristic of the apparatuses, systems and methods described herein is related to their ability to achieve high yields of the proteins, polypeptides or biologically produced polymers and small molecule compounds, while tolerating low passages of the desired compound through the filtration membrane. In various embodiments, the apparatuses, systems and methods described herein can improve the low yields related to the low passages of a desired compound by the way in which the system flows diafiltration solution through the system.

In various embodiments, the process can introduce diafiltration solution, including water or another diluent, into the last stage of the system as illustrated in Fig. 3 (stage 3), where the diafiltering solution combines with the retentate arriving from the previous stage. A portion of the desired compound then passes through the membrane, and this permeate, for example, a dilute solution of protein, can then be pumped into stage 2, and as a diafiltering fluid, be combined with the retentate arriving from stage 1. This mixture entering stage 2 can thus contain more of the desired compound than if fresh diafiltering solution had been added instead. The permeate from stage 2 can then be pumped into stage 1 and combined with the feed from the MF feed tank. Because the protein is in the feed and is also carried over by backfed permeate from stages 2 and 3, a permeate product greatly enriched with product compound, for example protein, can now leave the system as stage 1 permeate, and be removed from the system.

An additional method of controlling the product purity can result from providing diafiltration solution at the different entry points in the system. One way to gain the highest level of purity would be to operate in a strict counter-current mode with all of the diafiltration solution entering in the last stage as described in the previous paragraph. If the level of purity required is lower, the diafiltration solution can be introduced into one or multiple stages and permeate removed from the stages as desired to achieve the purity desired. The purity control procedure can combine two methods of diafiltration previously used as individual processes, but in a combined way not previously used before, in order to obtain "custom" levels of purity.

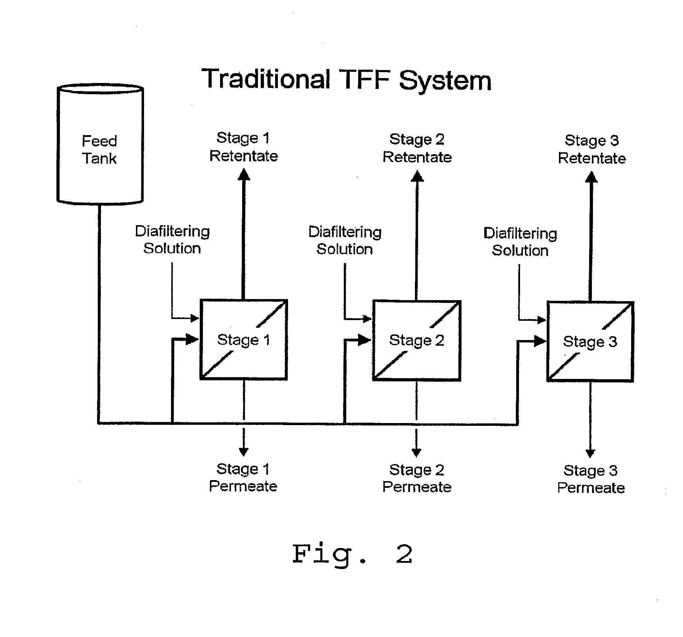

Figs. 2 and 3 demonstrate the differences in the flow between a traditional MF machine and a TFF microfiltration apparatus/machine/system described herein. In various embodiments, it is at least the differences in the flow of the diafiltration water that can contribute to obtaining higher yields relative to previous systems and methods.

Fig. 2 is a simplified figure illustrating the flow of diafiltration solution, including water or other diluent, in a traditional filtration system. The diafiltration solution enters each stage, and exits as permeate, carrying, for example, a protein with it. In this figure, specifically note the limited pathway possibility for permeate flow. The permeate only has the option for being removed from each stage, and not being introduced into any other stage. The end result is a machine/process that has little flexibility in terms of control and processing capability.

Figs. 3-5 exemplify filtration systems of various embodiments of the present invention that commonly include a plurality of fluidly-interconnected filtration modules including a first module and multiple subsequent modules. Each module is configured to route received feed material and diluent adjacent a filter to provide permeate and retentate. In each system, the first module receives feed at an inlet side from outside the system and the subsequent modules receive retentate from a preceding module within the system as feed material. In each system, at least one of the modules has a permeate withdrawal flow line for withdrawing permeate from the system, and multiple permeate withdrawal lines can be provided to blend permeate withdrawn from the system to a desired level of purity or other properties. A plurality of the modules have a return permeate flow line configured for recycling or returning permeate to an inlet side of the same module and or a permeate flow line for backfeeding permeate from a module to an inlet side of a preceding module within the system.

Fig. 3 illustrates an embodiment of the hydraulic path of a system/apparatus of the invention. The system exemplified in Fig. 3 has diafiltering lines and permeate recycle. The feed and large particles in the retentate can follow the path from the MF feed tank to the various stages, for example, stage 1 to stage 2 to stage 3. Permeate flow lines 31, 32 and 33 are shown in Fig. 3 for stages 1, 2, and 3, respectively. Permeate in lines 32 and 33 connect with permeate recirculation lines 321 and 331, respectively, which directs permeate to a previous stage of the system. Permeate lines 32 and 33 also connect with tap lines 322 and 332, respectively. The diafiltration solution can be added at multiple points in the system, with the protein/molecule of interest being "picked up" by the solution and washed into the earlier stages until it finally exits the system as stage 1 permeate. The feed and diafiltration paths flow in opposite directions. Purity can be selected and controlled by removing permeate from the different stages as well as by introducing the diafiltration solution into the different stages at rates needed and selected to achieve the level of purity desired. It will be noted that depending upon the level of purity desired for a molecule being purified, the permeate can be optionally removed at multiple points in the system, for example, from stage 3, stage 2 or stage 1. In various embodiments, the system can be adapted to optionally remove retentate or cell paste at multiple points in the system, for example, from stage 3, stage 2 or stage 1. The permeate tap lines 322 and 332 can be used to draw or pull off controlled amounts of permeate at one or more of the downstream stages. The drawn off permeate(s) can be used in blending operations with other permeate streams of the system, such as permeate drawn from the first module and or other subsequent stages, such that the overall purity of finished products can be adjusted to desired values using the TFF system.

The systems presented in Figs. 3-5, unlike the traditional system of Fig. 2, have the capability to allow control of flux, net permeation rate, yield and purity independent of one another.

Fig. 4 illustrates a schematic of an embodiment of the TFF system. The schematic is intended to provide a description of a system having multiple alternative pathways depending on the desired yield and purity of a desired product. As such, one of skill in the art will understand that many combinations of the pathways, recycling of liquids and additions of solutions are possible depending on the desired end result. It is not intended that the invention be limited to any single pathway, system or process in using the system described herein. It will also be understood that pathways presented in the schematics can actually comprise, for example, piping or conduits for flow of desired fluids.

It should be understood that in various embodiments, all or only a part of the system and its flow pathways can be used, depending upon the desired end-result, in terms of yield, quality, or economy of process. In addition, to the enumerated features of the flow pathway, various embodiments can comprise pumps, valves and other devices for regulating and controlling flow of solutions of interest. In various embodiments, the product of interest can be collected from a single point or multiple points in the system.

It should be understood to those skilled in the art that the optimal operation of the system relies on knowledge of how a particular feed material and product compound behaves under various operational conditions and that this knowledge is normally gathered through pilot- and production-scale studies.

According to various embodiments a filtration system of Fig. 4 can be provided comprising: a first filtration module 30 with a system inlet side configured to receive a feed stream, where module 30 can comprise a first inlet side and a first outlet side, the first inlet side being in fluid communication with the system inlet (for example, line 70); a second filtration module 32 comprising a second inlet side and a second outlet side, the second inlet side being in fluid communication with the first inlet side (for example, line 72); and a first interruptable bypass configured to define an interruptable fluid communication between the second outlet side and the first inlet side (for example, in line 63); and a system outlet, for example, 45 or 47 in fluid communication with each of the first outlet side and the second outlet side. In various embodiments, a system outlet from the second filtration unit can remove permeate from the system rather than recirculate the permeate between filter modules.

The TFF system of Fig. 4 can comprise diafiltration solution entry points 15, 17, 19, 21, 23, and 25. Some of the entry points can permit flow of diafiltration solution into the inlet side of the TFF modules. These entry points for the diafiltration solution can be used singularly or in combination, depending on the desired quality of the product of interest. There can be a single or multiple sources for the diafiltration solution. There can also be pumps in the system that pump the diafiltration solution from its source to the appropriate entry point into the system.

A TFF module (for example, 30, 32, 34, or 36) can contain any type of TFF technology (for example, polymeric, ceramic, stainless type material regardless of configuration type including, but not limited to spiral, tubular, hollow fiber, flat-sheet, etc.). A TFF module (for example, 30, 32, 34, or 36) can also contain a device to create tangential flow such as (but not limited to) a pump or any other such device that can create tangential fluid flow across the retentate side of the membrane. Alternatively, the tangential flow can be created outside the filtration module. This tangential flow can provide both pressure and assist separation and purification within the TFF machine of Fig. 4.

Purification and permeate hydraulic flow control streams are represented by 45, 47, 49, 51, 53, 55, 57, 59, 61, 63, and 65. The use of the streams will depend on whether the permeate is to be recirculated into the same filter module, pumped into a previous, or collected as product from the system.

The system of Fig. 4 can provide, inter alia, at least one of several properties including but not limited to, the ability to control the flow of the feedstream to be purified (or separated) through the system in a controlled and/or calculated way, the ability to purify or separate each stream (both retentate and permeate) or cell paste to a desired degree with accuracy and control, and/or the ability to run a purification (or separation) of a feedstream in a controlled manner regardless of the hydraulic filtration (or separation) rate from stage to stage. The system can reduce impurities in a controlled and efficient manner, eliminate other purification (or separation) steps, and perform a highly efficient purification (or separation) of multiple streams and products (or byproducts) with one system in an enclosed environment thereby protecting the safety of workers.

Referring to Fig. 4, the solution to be purified (or separated) 12 can enter into the feed tank 10. The initial capability to dilute or use a diafiltration solution for purification or to assist filtration is through line 15. Feed tank 10 can be adapted to have the capability to receive some or all of the retentate stream from the system in order to re-process, purify, or separate this partially processed stream further. There can be two exit points from the feed tank 10, a collection line can capture any processed material 40 from feed tank 10 as well as feed line 16 for entry into the first module. Material from line 40 is referred to in the figure as byproduct, however, in various embodiments, such material can also be a product of interest. Feed from line 16 can then be combined with a diafiltration solution 17 at a controlled rate to provide purification and/or to assist the separation in the system. The combination of material from 16 and diafiltration solution from 17 produces a retentate or feed that can enter the system via line 70.

The feed from line 70 can enter the first stage of the TFF indicated as a TFF module 30. The TFF module 30 can contain any type of TFF technology (for example, a polymeric, a ceramic, a stainless type material regardless of configuration type comprising spiral, tubular, hollow fiber, flat-sheet, etc.). In various embodiments, a diafiltration solution can be flowed through line 19 into TFF module 30. TFF module 30 can have an inlet side and an outlet side, as can all TFF modules used in the system. Line 63 can flow filtered material (for example, permeate) from TFF module 32. This permeate can be used to purify and/or assist separation of the feed material in TFF module 30 and can be added to TFF module 30 at a controlled ratio in order to control the purification and separation process. Purified and separated material that is produced from TFF module 30 can leave the machine through permeate product line 45, and can be recycled back into the inlet/feed side of TFF module 30 via line 65, and which is used, for example, to control overall purification and separation of the feed from feed tank 10. The exit point out of the retentate side of TFF module 30 can provide partially separated and purified retentate that can be sent to other TFF modules in the machine.

In various embodiments, partially purified and separated retentate or feed from TFF module 30 can enter TFF module 32 through line 72. TFF module 32 can contain any type of TFF technology (for example, polymeric, ceramic, stainless type material regardless of configuration type, for example, spiral, tubular, hollow fiber, flat-sheet, etc.). In various embodiments, a diafiltration solution can be flowed through line 21 into the inlet side of TFF module 32. Line 59 can contain filtered material (permeate) from TFF module 34. This permeate can be used to purify and/or assist separation of feed material in TFF module 32 and can be added to the inlet side of TFF module 32 at a controlled ratio in order to control the purification and separation process. Purified and separated material that is in the permeate from TFF module 32 can leave the system and be collected through the permeate product line 47, be recycled back into TFF module 32 via line 61 to control overall purification and separation of the feed from feed tank 10, or sent to TFF module 30 via line 63. If a permeate is leaving the system, it can leave directly from TFF module 32 via line 47, or can leave via an interruptable bypass (not illustrated) between TFF module 32 and TFF module 30. The interruptable bypass can be configured to define an interruptable fluid communication between the outlet side of TFF module 32 and the inlet side of TFF module 30. Line 61 can be used to control line 72 at TFF module 32 in an inverse correlation, i.e., line 72 flow can be decreased with an increase in line 61 flow.

In various embodiments, the system can comprise an interruptable bypass (not illustrated) that can be configured to define an interruptable fluid communication between the inlet side of TFF module 32 and the inlet side of TFF module 30. The interruptable bypass can be used to remove retentate flowing between TFF module 32 and TFF module 30 from the system for collection of product in the retentate. An interruptable bypass can be represented by feature 42, shown on the nth stage only, but it also can be provided with any preceding module of the system such as modules 30, 32, and 34.

The partially purified and separated retentate or feed from TFF module 32 can enter the inlet side of TFF module 34 through line 74. TFF module 34 can contain any type of TFF technology (for example, polymeric, ceramic, or stainless steel type material regardless of configuration, for example, spiral, tubular, hollow fiber, flat-sheet, etc.). Although not illustrated in Fig. 4, in various other embodiments partially separated and purified retentate from any one or more of modules among TFF module 30 through the (nth-2) stage module in the machine is distributed to one or more downstream TFF modules in the machine in addition to or in lieu of the immediate downstream TFF module thereto, and excluding upstream modules.

In various embodiments, a diafiltration solution can be flowed through line 23 into the inlet side TFF module 34. Line 55 can contain filtered material (for example, permeate) from TFF module 36. This permeate can be used to purify and/or assist separation of the feed material or retentate in TFF module 34 and can be added to the inlet side of TFF module 34 at a controlled ratio in order to control the purification and separation process. Purified and separated material that is produced from TFF module 34 can leave the system and can be collected through the permeate product line 49, be recycled back into TFF module 34 via line 57 to control overall purification and separation of the feed introduced from retentate line 74, or be sent to TFF module 32 via line 59. If a permeate is leaving the system, it can leave directly from TFF module 34 via line 49, or can leave via an interruptable bypass (not illustrated) between TFF module 34 and TFF module 32 The interruptable bypass can be configured to define an interruptable fluid communication between the outlet side of TFF module 34 and the inlet side of TFF module 32. Line 57 can be used to control line 74 at TFF module 34 in an inverse correlation, i.e., i.e., line 74 flow can be decreased with an increase in line 57 flow.

In various embodiments, the system can comprise an interruptable bypass (not illustrated) that can be configured to define an interruptable fluid communication between the inlet side of TFF module 34 and the inlet side of TFF module 32. The interruptable bypass can be used to remove retentate flowing between TFF module 32 and TFF module 34 from the system for collection of product in the retentate.

The exit point out 76 of the retentate side of the TFF module 34 can contain partially separated and purified retentate to be sent to the other TFF modules in the machine (except TFF module 30 and 32 or other preceding modules within the system).

The partially purified and separated retentate or feed from TFF module 34 can enter TFF module 36 through line 76. TFF module 36 can contain any type of TFF technology (for example, polymeric, ceramic, or stainless type material regardless of configuration, for example, spiral, tubular, hollow fiber, flat-sheet, etc.). In various embodiments, a diafiltration solution can be flowed through line 25 into TFF module 36. Because the TFF machine illustrated in Fig. 4 may have an infinite theoretical number of stages (or additional TFF modules), there can be filtered material (for example, permeate) from additional TFF modules. This permeate can be used to purify and/or assist separation of the feed material in TFF module 36 and can be added to TFF module 36 at a controlled ratio in order to control the purification and separation process. Purified and separated material that is produced from TFF module 36 can leave the system through permeate line 51, be recycled back into TFF module 36 via line 53 to control overall purification and separation of the feed from retentate line 76, or can be sent to TFF module 34 via line 55. Line 53 can be used control line 76 at TFF module 36 in an inverse correlation, i.e., line 76 flow can be decreased with an increase in line 53 flow. Exit point(s) out of the retentate side of the TFF module 36 can be line 78 and/or line 42. This material can be partially separated and purified retentate that can be sent to either i) additional TFF modules in the machine (since the machine can have infinite stages), ii) out of the machine via line 42, or iii) back into feed tank 10 for further processing via line 78. Line 78 returns retentate to the feed tank 10 from module 36, or any other nth stage module. The retentate leaving TFF module 36 should not go to TFF module(s) 30, 32, or 34. All permeate and retentate flows exiting the system can be controlled in a calculated manner as part of an overall strategy to control the overall purity and separation process in the TFF machine shown in Fig. 4. If a permeate is leaving the system, it can leave directly from a TFF module, or can leave via an interruptable bypass between TFF modules. The interruptable bypass can be configured to define an interruptable fluid communication between the outlet side of one TFF module and the inlet side of another TFF module.

Diafiltration solution entry points (for example, at 15, 17, 19, 21, 23, or 25) can be determined to obtain various ranges and/or quality of diafiltration (or washing/extracting/purification) solutions. The ratios and/or flowrates of each of these streams in relation to the feed can be determined for various operating ranges, as well as, to obtain a desired purity control in the process using the filtration system.

As noted above, the TFF machine shown in Fig. 4 can have an infinite number of stages in its design to control the overall purity and separation process of the products and byproducts of the machine. These additional stages can have a similar setup in regards to permeate or retentate recirculation options.

The above description provides for removing permeate and retentate at various stages in the process. It should also be noted that a retentate product, for example cell paste, can be removed from the various filtration modules if a product of interest is retained in that portion of the filtration system.

It should be understood that in various embodiments additional piping (for example, conduits) and TFF modules can be added. From the above description, it would be clear to one of skill in the art, how to process a fluid using the additional TFF modules.

Features 40 and 42 can provide dual operation of batch and/or continuous-flow modes. Lines 40 and 42 can be used to collect product as deemed appropriate. This flexibility in design can be used to control the purity and product quality of the final product out of the machine and/or can allow a manufacturing plant to control the speed at which the machine can make a product.

According to various embodiments, the piping/conduits/flow lines (for example, 40, 42, 78, 72, 74, 76, 51, 53, 55, 49, 57, 59, 47, 61, 63, 65, & 45) can be used at various operating ranges, for example, flow rate or pressure, in order to a) control hydraulic imbalances in the machine that can occur due to purity control capabilities that may be used to make differentiable products with the system; b) control the purity of the final products produced with the system; and/or c) control the efficiency and speed at which differentiable products can be made by the system. For example, hydraulic imbalances can be controlled by flow from 30 into 32, 34 and 36 in order to control yield and purity. These modules can have a fixed volume. If one cannot control flow out then one may need to control the flow of lines 72, 74, 76 into the modules, for example, lines 53, 57, 61 and 65 can control the flow of 72, 74 and 76. According to various embodiments, permeate can be fed from the above system into a second system.

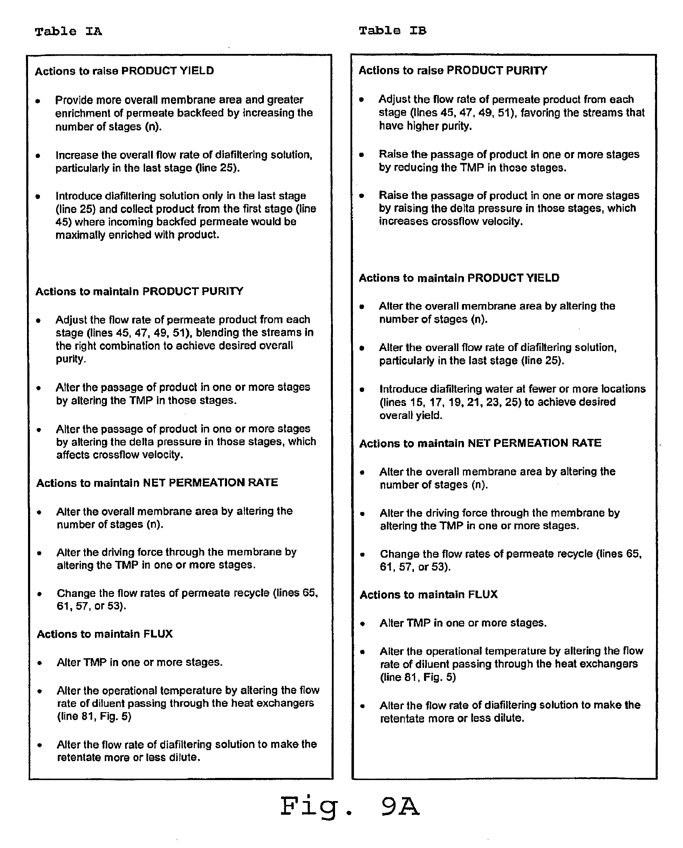

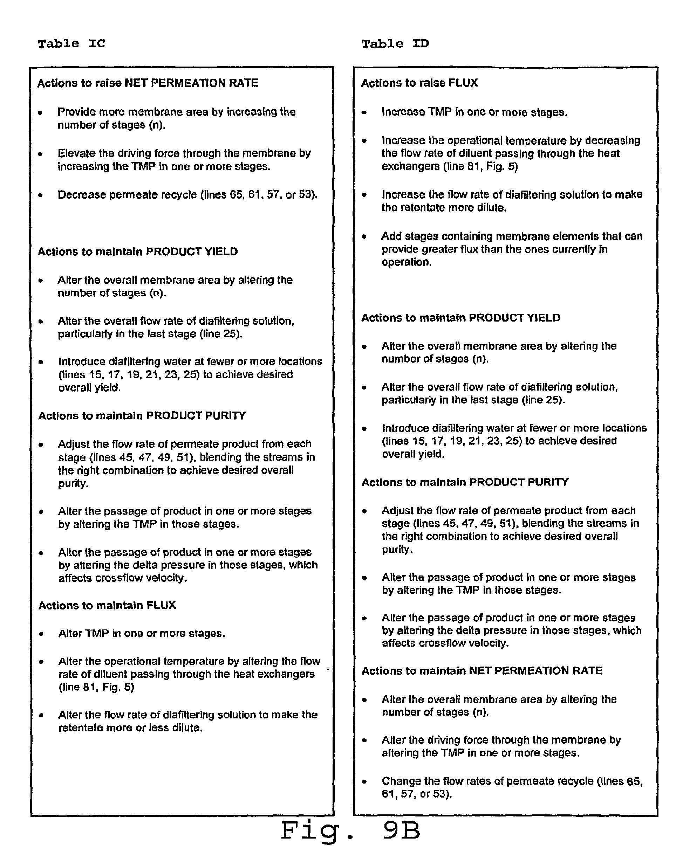

The system of Fig. 4, and Fig. 5 described in more detail below, can be operated to independently manipulate any one of overall product yield, product purity net permeation rate, and flux, where the product is collected from the permeate side. Referring to Figs. 9A and 9B, the Tables IA-ID provide exemplary illustrations on how one of the parameters of overall product yield, product purity, net permeation rate, or flux, can be raised while maintaining the other three variables constant or at least approximately constant. Table IA-ID are formatted such that an exemplified kind of action used to raise a given variable is indicated in the upper box of a given column, and the concurrent kinds of actions that can be taken to maintain the other three variables constant are indicated in the underlying sections of the same column. The degree of adjustment needed for each listed kind of action indicated in Tables IA-ID can be determined empirically on the system to learn how a particular feed and product will respond to various sets of process conditions encompassing at least the four mentioned above. For example, as exemplified in Table IA of Fig. 9A, for a given product, yield can be raised by providing more overall membrane area, increased addition of diafiltering water, and or introducing diafiltering solution only at the nth stage and collection of product from the first stage, and the respective counter-measures can be taken to maintain purity (for instance, adjust the flow rate of permeate product from each stage and blending the streams, and so forth), net permeation rate (for instance, alter overall membrane area by altering number of stages, and so forth), and flux (for instance, alter TMP in one or more stages, and so forth). One or more of the exemplary adjustments can be applied that are listed under each parameter heading in the tables. Those skilled in the art will understand that empirical information and knowledge can be acquired in advance, for example, through pilot studies or production-scale studies conducted on the system.

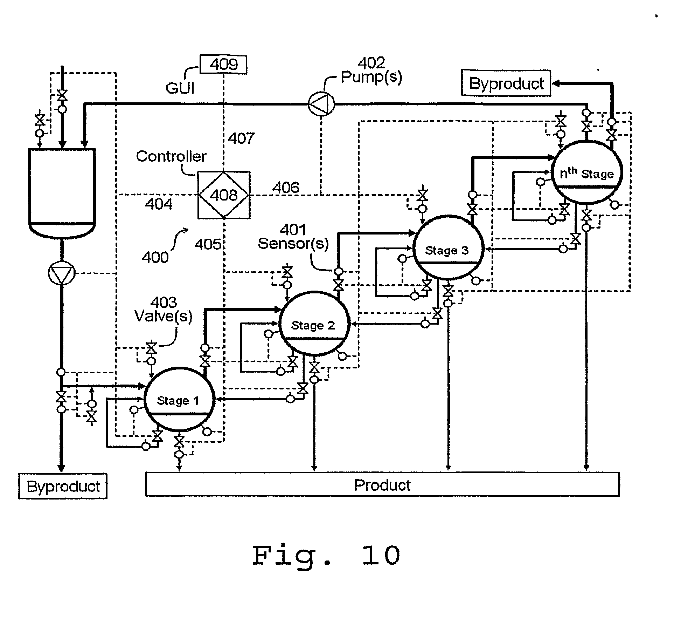

These operational controls can be controlled manually or in automated manner, or in part with both, to implement the operational examples exemplified in Tables IA-ID. Referring to Fig. 10, the embodiment of Fig. 4 is illustrated including an exemplified control system 400 and components integrated therewith. The control system 400 allows control over relevant operational parameters of the system in a coordinated automated manner. A plurality of sensors 401 can be provided for acquiring data (e.g., flow rate, pressure, concentration, pH, conductivity, temperature, turbidity, ultraviolet absorbance, fluorescence, refractive index, osmolarity, dried solids, near infrared light, or Fourier transform infrared light) about fluids as they flow through various parts (e.g., lines) of the system and through the filtration modules. The system also has a plurality of pumps 402 and valves 403 for pumping and regulating flow of the fluids through the system. Only several such components are exemplified in Fig. 4 for sake of clarity in the illustration. Persons skilled in the art will appreciate other convenient and useful installation sites for such components within the system for implementing the teachings disclosed herein. The control system 400 can be an electronic data processing network, including communication links 404, 405, 406, 407, etc., indicated by hatched lines, between the control components 401-403, etc., and a controller 408, can be provided capable of at least receiving, processing, recording, and transmitting data and signals associated with the operation of said pumps, valves, and sensors, wherein the recorded data collected during a flow filtration process is sufficiently comprehensive to control the flow filtration process. These communication links can be hardwired, wireless, or combinations thereof, and include appropriate hardware and software for transmitting, receiving and processing signals as applicable, as will be appreciated by persons skilled in the art. The controller 408 can also convert the signals received from the process control devices into displayable readings of flow rate, pressure, concentration, pH, conductivity, temperature, turbidity, ultraviolet absorbance, fluorescence, refractive index, osmolarity, dried solids, near infrared light, or Fourier transform infrared light, etc. which can be displayed at the component (via return signal communication), on the controller, and/or on a graphical user interface 409 including a computer display monitor (not shown) linked to the controller 408 for inter-communication. The graphical user interface 409 also can be used to communicate with the controller 408 regarding management and implementation of algorithms relevant to automating the operational parameters adjustment schemes exemplified in Figs. 9A and 9B.

As previously indicated, for a given set of process conditions and equipment set-up, the manufacturing system can be pre-sampled to empirically learn how a particular feed and product will respond to various sets of process conditions applied on the system exemplified herein. For example, such empirical studies can be used to develop a predictive model, which embodies mathematical algorithms, of the relationships between sensed parameter values, a desired adjustment to alter the value of one operational parameter, and choice and degree of adjustments to be made at the other operational parameters to maintain them constant during adjustment of the other parameter. To implement such a predictive model, the controller may comprise a programmable logic controller (PLC) having access to computer code, embodied in microelectronic hardware mounted on a motherboard or the like and/or in software loaded on a remote computer (not shown) in communication therewith via the graphical under interface 409. Commercially available PLC modules may be modified to support these functionalities based on the teachings and guidance provided herein, including the information in Figs. 9A and 9B. The controller system can have both hardware components and software, which may be adapted to develop and implement such algorithms for process control as exemplified herein.

It should be noted that the systems described above and elsewhere in the application are not limited to microfiltration. The system capabilities can be used with any crossflow filtration, where one desires to separate multiple components and may need to control purity and speed of manufacturing of the final product or byproduct.

In various embodiments, two or more of the systems/apparatus/machines described herein can be combined to create a larger, more complex system/apparatus/machine. The system can be connected in series to one another.

According to various embodiments, the system can be a microfilter system. A filtration module, for example a TFF module (30, 32, 34, or 36 in Fig. 4) can use a filter element of defined pore or molecular size range that can be commercially purchased. Operational conditions for this module, for a desired result, can be determined by one of skill in the art.

Fig. 5 illustrates another embodiment of the system. The numbers identifying features in Fig. 4 identify similar features in Fig. 5. Fig. 5 also illustrates the system configured to yield a product from a single TFF module following recirculation through additional units. Additionally, Fig. 5 illustrates a system configured for independent cleaning of the filtration modules (lines 31, 29), use of heat exchangers (26, 27) and a cooling bleed-off piping (line 82). These characteristics of the system can be equally applied to the configuration of Fig. 4.

In various embodiments, one can control the temperature of a TFF system by adding a cooling bleed off line for flow of diafiltration cooling solution. A diafiltration solution 81 can cool a TFF system by first passing through heat exchangers 26 and 27. If desired, additional heat exchangers can be placed in the system. The diafiltration solution can then enter TFF filtration module 34 via line 83. In various embodiments, rather than the diafiltration solution entering the TFF module after passing through the heat exchangers, the solution is bled off through line 82 and fresh diafiltration solution is added at 23. In this way, the temperature can be better controlled to preserve products whose properties may be temperature sensitive or to impose a desired temperature to thermally treat a product. With this feature, one can control the purification and separation in the TFF system and control the temperature of the system during operation. These internal heat exchange (cooling) configurations make it easier to control operating temperature of the process, which, in turn, facilitates control over yields, capacity and/or product purity in a consistent and reproducible manner.

In various embodiments, the temperature of various fluid streams in the system can be regulated by adjusting the temperature and relative flow rate of a diafiltering solution entering the system (15, 17, 23, and 83), where the diafiltering solution temperature can be adjusted by blending hot and cold water to make up the solution before it travels to the system.

In various embodiments, the pH or conductivity of various fluid streams in the system can be regulated by injecting a concentrated acid, base, or salt solution, where a metering pump or an automated valve may be used to modulate the injection rate of the solution and where such a pump or valve would receive a control signal tied to the output signal from a pH or conductivity sensor via a central controller (Fig. 10).

In various embodiments, as shown in Fig. 5, line 29 at TFF module 32 and line 31 at TFF module 34, can be added to the system. These lines can be used to independently deliver cleaning chemical at a known concentration and temperature directly to all three TFF modules (TFF module 30 can receive cleaning chemical from line 70) from tank 10. In conventional systems all of the cleaning chemicals must pass through the first module, where they lose potency, prior to reaching subsequent modules of the system, resulting in poorer cleaning and poorer separation/purification from TFF module 32 and 34 during the subsequent product run. Without this modification inconsistent results were obtained.

In various embodiments, the process can be used to recover an enzyme from a fermentation broth. The fermentation broth is transferred to a holding tank where the pH of the broth may be adjusted if necessary. The fermentation broth is then pretreated for transfer to either a TFF microfiltration unit or another separation module, such as a rotary drum vacuum filtration (RDVF) module. When the broth is to be transferred to a TFF unit, it is pretreated by adjusting the dissolved solids to about less than 10% by the addition of water, and the diluted broth passes through a screen to remove larger particles, typically above 350 microns, prior to entry into the TFF microfiltration unit. When the broth is to be sent to a RDVF module, it typically is pretreated with an adsorbant and/or flocculant. Prior to transfer to either the TFF unit or the RDVF module, the broth is held for a period of time to allow for proper mixing of all components.

TFF separation is accomplished by selecting and setting, for example, TMP, concentration ratio, and a diafiltration ratio. The diafiltration solution may be injected into the inlet side entry point of any TFF module in the TFF unit. The diafiltration solution may comprise the separate streams entering each stage or the counter-current stream of permeate traveling from one stage to another. The diafiltration solution may extract components multiple times as it is circulated through the TFF unit modules, and by controlling where the permeate exits the TFF unit, the purity of the recovered enzyme can be adjusted. An ultrafiltration unit may be operated in parallel with the TFF unit to concentrate permeate from the TFF unit. For example, enzymes recovered from the ultrafiltration unit may be formulated without additional filtration.

Alternatively, when the treated fermentation broth is sent to an RDVF module for cell separation, the temperature and feed rate are selected and the broth is fed to the drum in about two to three cycles with the addition of water sprayed onto the drum to wash the soluble enzyme through the drum filter. An ultrafiltration unit can be run in parallel with the RDVF process to concentrate the solution from the RDVF module. The pH of concentrated product is then adjusted, if necessary, and the concentrate is sent to a filterpress to clarify the solution and reduce contaminants found in the operating environment, for example, ambient microbes.

### Examples

The following examples merely represent various embodiments of the invention. The examples are not intended to limit the invention in any way whatsoever.

### Example 1

A set of experiments was conducted that demonstrated the advantage of the TFF microfiltration system during the microfiltration purification of glucoamylase enzyme from a fermentation broth composed of a mixture of corn steep solids, salts and a carbon nutrient. In the experiment, a Koch membrane module (Koch Systems, Wilmington, Mass.) Model MFK-601-FYT (8338) was used in this Example. A similar module was used in Examples 2 and 3 except for a smaller diameter (3838). The production host was *Aspergillus niger.* At the end of fermentation, the pH of the broth was adjusted to about pH 3.3 with sulfuric acid; 3.5% bentonite was added to the broth; and the broth was held for a minimum of 12 hours before beginning the microfiltration process. No water or diafiltration solution was added to the broth before the start of the clarification process, and no flocculants or filter aids were used for these experiments.

Compared to a traditional TFF system, the system of the application can achieve greater product yield at conventional TMPs. The traditional system (Fig. 2) and the TFF microfiltration system (Fig. 3) were set to operate at the same operating conditions. In both cases, the inlet side feed pressure was set at 3.5 bar, and the inlet side exit pressure at 1.5 bar. The membranes used were spiral configuration, with a nominal pore size of 1.2 microns. The diafiltration ratio was set at 3.0 (diafiltration solution to feed), the concentration ratio was set at 0.7 (cell paste flow rate out to feed), and the temperature set at 30° C.

The traditional microfilter had an average transmission of 49.3%, (initial transmission at 50%, and 48% at the end of the run), and an overall yield for the run of 73.5% for the experiment.

The counter-current TFF system as described herein provided a significant benefit over the traditional system. The system had an average transmission of 41% (45% early in the run and 38% at the end), and overall yield of 94.2%. This yield difference can take a process that would not be developed and make it a possible processing option due to the many characteristics described earlier in the application.

### Example 2

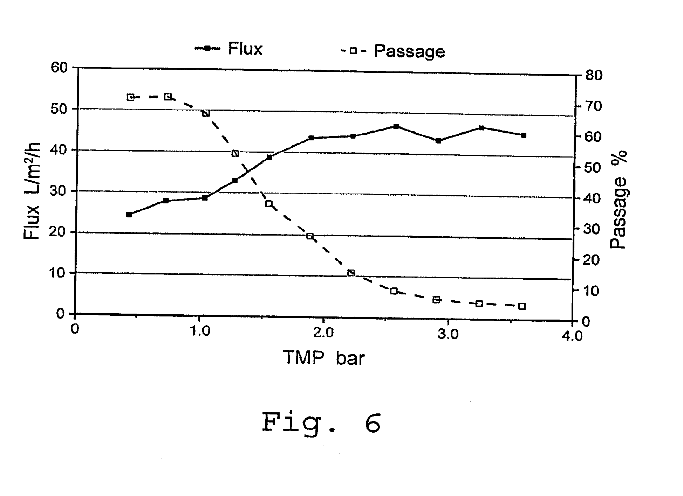

Results in Fig. 6 was obtained with a conventional TFF system using a microfiltration membrane and a feed consisting of *B. subtilis* broth and protease enzyme. The results provide information concerning altering TMP and the effect on flux and passage of material through the membrane in a filtration unit (a simplified version of Fig. 2 with one stage).

Fig. 6 illustrates that for a product using low TMP pressures where traditional MF machines operate, the passage of the protein is sufficient to be able to operate up to about 0.9 bar TMP and have passages of 70% or above, with a flux rate of about 28 L/m²/h (lmh). The TFF microfiltration apparatus/system described herein, however, could operate at a higher TMP of about 1.5 bar, and despite a passage of about 40%, could achieve over all yield of 90-92%, (see Example 1).

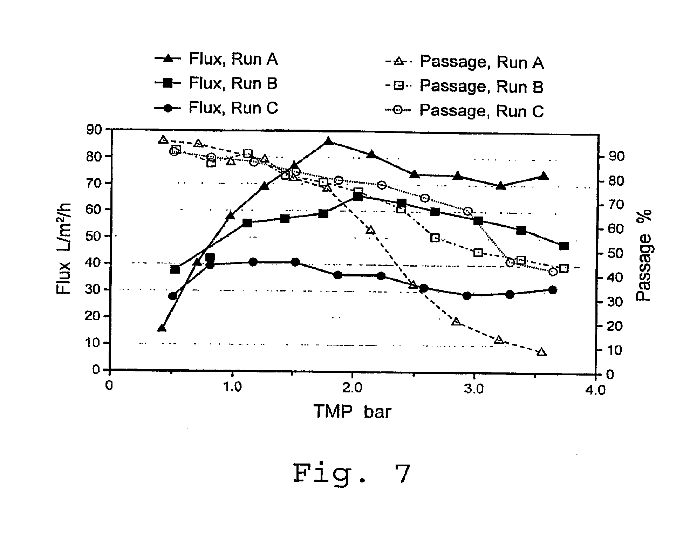

Fig. 7 provides another illustration of how the counter-current machine can provide a benefit. It shows data from three separate experiments demonstrating the impact of TMP vs. flux and passage on *B, licheniformis* broth containing alpha-amylase enzyme. Each experiment was performed at a different crossflow velocity (delta P was 0.7, 1.0, and 1.5 bar, in A, B, and C, respectively). The TFF microfiltration machine could operate at 2 bar TMP and have passages in the area of 70-75% and an overall yield of 97-99% and flux rates of 85 lmh. An engineering model was used to predict yield by combining empirical data and mass balance equations. The data illustrates that one can control purity, yield and flux of a desired component independent of one another. In contrast, a traditional machine would need to operate in the 0.6 bar TMP range at a flux of 40 lmh, with passages around 90%, and an overall yield of 90-92%. As such, this data suggests that the TFF microfiltration invention fluxes can be >2x at the higher TMP, as well as have an overall yield of 5-7% greater than a traditional machine.

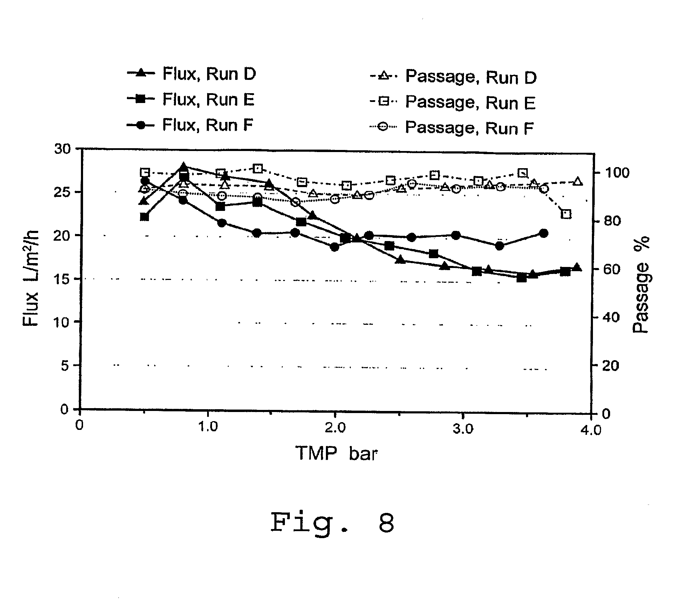

Fig. 8 demonstrates an instance where the TFF microfiltration invention may not have the same level of benefit described above relative to a traditional machine. The product tested in this case demonstrates that both flux and passage are high at the lower TMPs of a traditional machine, so very little to no advantage in terms of flux and passage would be obtained. However, even in this case the TFF microfiltration can still have the benefit of delivering higher product purity. By operating at the higher TMPs, a more dense boundary layer forms on the surface of the membrane thereby causing the rejection of a greater number of impurities.

### Example 3

Table 1 lists several products that were obtained using the systems and methods described herein. These products made from either bacterial or fungal broth represent enzymes, each with unique properties although some may share similar enzymatic function and are said to be of a particular type, for example alpha-amylase #1 through #4. Various parameters used in obtaining the products are also listed herein.

**Table 1. Products and Production Parameters**

| Product | Delta P (bar per element) | Inlet side feed pressure (bar) | Inlet side exit pressure (bar) | Diafiltration-to-broth ratio | Concentration ratio |
|---|---|---|---|---|---|
| Alpha-amylase #1 | 0.5-1.5 | 1.5-4.5 | 0.5-1.5 | 2.0-6.0 | 0.3-1.0 |
| Alpha-amylase #2 | 0.5-1.5 | 1.5-5.5 | 0.5-3.0 | 2.0-6.0 | 0.3-1.0 |
| Alpha-amylase #3 | 0.5-1.5 | 1.5-4.0 | 0.5-2.5 | 2.0-6.0 | 0.3-1.0 |
| Alpha-amylase #4 | 0.5-1.5 | 1.5-3.5 | 0.5-2.0 | 2.0-6.0 | 0.5-1.0 |
| Cellulase # I | 0.5-1.5 | 1.5-3.5 | 0.5-2.0 | 2.0-6.0 | 0.3-1.0 |
| Cellulase #2 | 0.5-1.5 | 1.5-5.5 | 0.5-3.5 | 2.0-6.0 | 0.3-1.0 |
| Glucoamylase | 0.5-1.5 | 1.5-3.5 | 0.5-1.0 | 2.0-6.0 | 0.3-1.0 |
| Mannanase | 0.5-1.0 | 1.5-3.0 | 0.5-1.0 | 2.0-6.0 | 0.3-1.0 |
| Peroxidase | 0.5-1.0 | 1.5-3.0 | 0.5-1.5 | 3.0-7.0 | 0.3-1.0 |
| Protease #1 | 0.5-1.5 | 1.5-5.5 | 0.5-3.5 | 2.0-6.0 | 0.3-1.0 |
| Protease #2 | 0.5-1.5 | 1.5-5.5 | 0.5-3.5 | 2.0-6.0 | 0.3-1.0 |
| Protease #3 | 0.5-1.5 | 1.5-5.5 | 0.5-3.5 | 2.0-6.0 | 0.3-1.0 |
| Pullulanase | 1.0-1.5 | 1.5-5.5 | 0.5-3.5 | 2.0-6.0 | 0.3-1.0 |

### Example 4

Batches of protease, cellulase, and amylase concentrate products were obtained as outlined below in Table 2 to compare properties of the enzymes using the TFF unit of an embodiment of the present invention for separation as compared to properties of the enzyme produced using RDVF or centrifugal cell separation (traditional methods).

### TFF microfiltration invention operating conditions.

The TFF microfiltration unit was equipped with spiral 1.2 micron nominal pore size, polyethersulfone (PES) membrane elements. Feed broths were adjusted to pH 5-7, depending on the product enzyme. The diafiltration ratio was set to 3 parts of water to 1 part feed, and diafiltering water entered from stage 3, but could enter at any of the points indicated in Fig. 3. The inlet side exit pressure had a setpoint of 0.5 bar, and the inlet side feed pressure was set at 3.25 bar. The temperature was set at 35 °C (acceptable range: 10-45 °C), and the concentration ratio was set at 0.6 to 1.0 depending on the feed rate. An ultrafiltration unit, running in parallel with the TFF microfilter, performed a 10-20x concentration of the microfilter permeate. Because the microfiltration process can provide a purer and less contaminated product stream compared to other methods of cell separation, the ultrafiltration process in turn gains an ability to produce purer and less contaminated concentrate, leading to less turbidity caused by colloidal particles, and thus, less need for a final polish filtration step.

### RD VF operating conditions.

In cell separation by RVDF, the broth pH was adjusted to 5-7, a diatomaceous earth (DE) and flocculant were added to the broth, and the drum of the RDVF was precoated with DE. The broth was then fed to the drum until the precoat was depleted, at which time the separation was shut down for cleaning and installing another precoat. Usually, 2-3 of these precoat cycles are performed to process one batch of broth. Water was added to the process in the form of spray onto the precoat during the separation to wash the soluble components (including the product) through the cake. The temperature of the cell separation can be held at either 15 or 50 °C to minimize contamination by ambient microbes since the filtration is open to the environment. An ultrafiltration unit, running in parallel with the RVDF, was used to concentrate the RVDF filtrate by 10-20x. Prior to formulation, the final ultrafilter concentrate is normally filter pressed to reduce the level of ambient microbe contamination that may have entered at the RVDF step and multiplied during the downstream process. In the TFF microfiltration process, this polishing step is normally unnecessary because the process streams aren't exposed to the environment enough to pick up contamination.

### Centrifuge operating conditions.

Cell separation can also be done by decanter centrifuge. At the start of this process, the feed broth was pH adjusted to 5-7 and treated with flocculant before it was fed into a centrifuge. The broth may be diluted with as much as 2 parts of diafiltering water before entering the centrifuge. There are two process streams coming out of the centrifuge: a clarified liquid (centrate) which contains the product compound and a sludge which contains primarily dewatered cells and other insoluble matter. It is possible to send the sludge into a second centrifuge along with more diafiltering water, adjusting its pH and adding more flocculant as needed, to extract more product compound into a second centrate to be combined with the first centrate. The centrifuges were operated at 3000-4000xg, depending on the feed rate. Centrate was sent to an RVDF to remove particulates that were too small to settle and partition with the sludge in the centrifuge. The operation of the RVDF was the same as that previously described except that no flocculant treatment of the centrate was needed before the centrate was fed to the drum. An ultrafiltration unit, running in parallel with the RVDF, concentrated the RVDF filtrate 10-20x. As in the RVDF cell separation process, the final ultrafiltrate normally requires a polish filtration to remove ambient microbial contamination before the ultrafiltrate can be formulated into a final product.

The batches of enzymatic products were compared using standard assays to measure activity levels and other composition thereof. The results are presented in Table 2, which is comprised of sub-Tables 2a-2c.

The data analysis of Table 2 illustrates there was a significant increase in purity for the TFF products versus the conventionally made products ("Conv."). The increases in activity relative to a selected other component indicate that there were fewer impurities in the TFF produced material (Table 2a). Likewise, the decreases in absorbance relating to lighter color indicate also fewer impurities in the TFF produced material (Table 2b).

Table 2. Final Product Data Comparison

**Table 2a. Greater Purity of Several TFF Product Concentrates¹**

| Metric Purity | Protease² | | Cellulase² | | Amylase² | |
|---|---|---|---|---|---|---|
| | TFF | Conv. | TFF | Conv. | TFF | Conv. |
| Activity/ Total Protein | 1.20 | 0.59 | 0.0363 | 0.0348 | 0.915 | 0.851 |
| Activity/ Nitrogen | 62.5 | 31.1 | 1.870 | 1.810 | 53.4 | 44.4 |
| Activity/ Carbon | 16.9 | 7.9 | 0.511 | 0.485 | 10.4 | 9.8 |
| Activity/ Sulfur | >771 | 501 | 18.5 | 15.7 | >430 | >410 |
| Activity/ Iron | 4.2 | 2.0 | 0.540 | 0.105 | 10.3 | 6.7 |
| Activity/ Phosphorus | 0.113 | 0.054 | 0.040 | 0.017 | 0.0101 | 0.0146 |
| Activity/ Ash | 189 | 112 | 162 | 25 | 29.0 | 35.3 |
| Activity/ Solids | 8.6 | 3.9 | 0.251 | 0.237 | 4.58 | 4.22 |

**Table 2b. Lighter Color of Several TFF Product Concentrates**

| Product | Sample Dilution Factor | Absorbance x Dilution Factor/ Activity | | | |
|---|---|---|---|---|---|
| | | 470 nm | | 520 nm | |
| | | TFF | Conventional | TFF | Conventional |
| Protease | 10 | 0.104 | 0.196 | 0.066 | 0.125 |
| Cellulase | 10 | 0.128 | 0.354 | 0.060 | 0.219 |
| Amylase | 10 | 0.0416 | 0.0829 | 0.0282 | 0.0495 |

**Table 2c. Description of Measurements**

| Measurement | Units | Description |
|---|---|---|
| Activity | mg/g, mg/ml, kU/g, or kU/ml | Enzymatic activity |
| Total Protein | mg/mL | Proteins, peptides, amino acids |
| Nitrogen, Carbon, Sulfur | weight % | Elemental composition |
| Iron, Phosphorus | Ppm | Elemental composition |
| Ash | weight % | Noncombustible components |
| Solids | weight % | Nonvaporizable components |
| Absorbance | absorbance unit | Degree of light blockage; darkness |

*Table 2 Notations*/*Definitions:* **1.** To make product concentrates, broths were clarified by either TFF microfiltration or conventional method (RVDF or centrifuge); and the resulting product streams were than concentrated by 10 k MWCO ultrafiltration to equivalent activity (difference within 10% of the greater). **2.** With formulation, including addition of stabilizers, these protease, cellulase, and amylase products, would be given the trade names, Purafect®, Multifect®, and Spezyme®, respectively.

Enzymatic activity was measured by manual or automated methods, depending on enzyme. The methods employed different substrates (e.g. carboxymethylcellulose) and buffers of varying salts and pH. The activity was ultimately determined colorimetrically using a Beckman DU640 spectrophotometer or a Konelab autoanalyzer (Thermoelectron). Total protein was determined by Biuret color reaction (Pointe Scientific reagents) using a Konelab autoanalyzer on an entire sample, which would include all proteins, polypeptides, and amino acids. Bovine serum albumin was used to create a standard curve. A quantitative "dynamic flash combustion" method was used with a Fisons EA1108 Elemental Analyzer to determine elemental nitrogen, carbon, and sulfur composition, which are reported in weight percent. A Perkin Elmer Optima 3200 Inductively Coupled Plasma Optical Emission Spectrometer (ICP-OES) was used for the analysis of inorganic elements. Samples were digested with sulfuric and nitric acid. Inorganic elements are reported in parts per million (ppm). Elements routinely tested include silver, aluminum, arsenic, bismuth, calcium, cadmium, cobalt, chromium, copper, iron, potassium, magnesium, manganese, mercury, sodium, nickel, phosphorus, lead, antimony, silicon, tin, titanium, and zinc. Silliker Laboratories (Chicago Heights, Illinois) analyzed samples for ash and moisture. Ash content was determined following the AOAC (Association of Analytical Chemists) Method 930.22. Total solids were determined by subtracting moisture weight percent from 100%, where moisture content was determined following AOAC Method 926.08. Color (dark appearance) was estimated by recording the sample absorbance at 470 and 520nm using a Beckman DU640 spectrophotometer. Samples were diluted to reach an absorbance within the instrument range (0-2.0) and spun to remove any particulates which might influence light scattering.

Changes in purity, as defined by change in the active to total protein, and absorbance readings for color, measured in the inventive TFF samples in comparison to the RVDF) or centrifuge conventional samples ("Conv.") as the benchmark are indicated in Table 3.

**Table 3. % Changes In Purity and Absorbance Readings for Color**

| **Purity Metric** | **Protease** | | | **Cellulase** | | | **Amylase** | | |
|---|---|---|---|---|---|---|---|---|---|
| | **TFF** | **Conv.** | **% Change** | **TFF** | **Conv.** | **% Change** | **TFF** | **Conv.** | **% Change** |
| Activity/ Total Protein | 1.20 | 0.59 | +103.4 | 0.0363 | 0.0348 | +4.3 | 0.915 | 0.851 | +7.5 |
| Activity/ N | 62.5 | 31.1 | +101 | 1.870 | 1.810 | +3 | 53.4 | 44.4 | +20 |
| Activity/ C | 16.9 | 7.9 | +114 | 0.411 | 0.485 | +5 | 10.4 | 9.8 | +6 |
| Abs (470 nm)/ Activity | 0.104 | 0.196 | -46.9 | 0.128 | 0.354 | -63.8 | 0.0416 | 0.0829 | -49.8 |
| Abs (520 nm)/ Activity | 0.066 | 0.125 | -47.2 | 0.060 | 0.219 | -72.6 | 0.0282 | 0.0495 | -43.0 |

As shown by the data results in Table 2 and Table 3, the products obtained by the TFF system and process exemplifying the present invention have greater purity as compared to the products obtained with the conventional system (e.g. RVDF) for the variety of different tested enzymes. In particular, the results in Table 3 show at least about 4% increases (improvements) in activity per total protein exhibited by the TFF samples in comparison to the conventional samples, with an even much larger improvement seen for protease (i.e, about 103%). Regarding the N and C metric values reported in Table 3, and without desiring to be bound to theory, it is thought that the greater activity/N of the TFF products may indicate greater removal of certain nitrogen-containing impurities, e.g. DNA, and the greater activity/C of the TFF products may indicate a greater removal of certain carbon-containing impurities, e.g. carbohydrate polymer, compared to the conventional products. While not desiring to be bound to theory, it is also thought that operating at a high TMP, as the invention allows without sacrifice to yield, can generate a gel layer that can act to exclude these types of molecules whose effective sizes are much larger than folded proteins. Testing of these TFF and conventional products also indicates that a significant difference in color exists between the average color of the RDVF product versus the counterpart TFF products, such as measured in absorbance units at 470 nm and 520 nm. The significantly lower absorbance per activity measured for the TFF products than the RDVF products, such as shown by the % change in values reported in Table 3, also indicates a significant increase in purity for the TFF products, and, while not desiring to be bound by theory, it is thought that these results indicate a large number of molecules which cause color have been removed in the TFF products relative to the conventional products.

In addition to color changes, TFF products may have enhanced odor and taste properties that allow them to be used in food and brewing applications where these characteristics are important.

It is expected that the products with conventional levels of purity made by the traditional processes will have a significantly greater number of impurities, as well as a higher number of microbial contaminants.

The products obtained by the methods and apparatuses of the invention can be collected after processing by the TFF machine. Products can then be concentrated and purified by traditional methods, packaged and sold.

Other embodiments of the present teachings will be apparent to those skilled in the art from consideration of the present specification and practice of the present teachings disclosed herein. It is intended that the specification and examples be considered as exemplary only and not be limiting of the claims. All cited references, patents, and patent applications are incorporated in their entireties herein by reference.
The following numbered paragraphs contain statements of broad combinations of the inventive technical features herein disclosed:-
1. A filtration system comprising a plurality of fluidly-interconnected filtration modules including a first module and multiple subsequent modules, wherein each module is configured to route received feed material and diluent adjacent a filter to provide permeate and retentate, the first module receiving feed at an inlet side from outside the system and the subsequent modules receiving retentate from a preceding module within the system as feed material, at least one of the modules having a permeate withdrawal flow line for withdrawing permeate from the system, and a plurality of the modules having a return permeate exit configured for feeding permeate back to an inlet side of a same or a preceding module within the system.
2. The system of paragraph 1, further comprising a control system configured for independent adjustment of one of product yield, product purity, net permeation rate, and overall flux while maintaining the three other variables approximately constant.
3. The system of paragraph 1, wherein the first and subsequent modules each have a permeate recycling line for returning permeate to the inlet side of the same module, and the modules subsequent to the first module further comprise a permeate recirculation line for backfeeding permeate to a preceding module within the system.
4. A filtration system comprising:
   a system inlet for receiving fresh feed from outside the system,
   a plurality of fluidly-interconnected filtration modules comprising a first module and multiple subsequent modules,
   wherein the first module comprises:
      an inlet side in fluid communication with the system inlet for receiving fresh feed, a diafiltration line for introducing diluent to the inlet side, a filter for selectively separating a species of interest from feed material comprising a mixture of fresh feed and diluent for providing permeate that passes through the filter to an outlet side of the module and
      retentate that does not pass through the filter, at least one permeate flow line for withdrawing permeate from the outlet side of the first module and optionally another permeate flow line for recycling permeate to the inlet side of the first module, and a retentate flow line configured to route retentate flow from the inlet side of the module to the inlet side of a subsequent module,
   wherein the subsequent modules each comprise:
      an inlet side in fluid communication with a retentate flow line of a preceding module, a diafiltration line for introducing diluent to the inlet side, a filter for selectively separating the species of interest from feed material introduced at the inlet side thereof, a permeate flow line configured for returning permeate flow from the outlet side of the module to the inlet side of the same module or for backfeeding permeate flow to a preceding module, another permeate flow line configured for withdrawing permeate from the system, and a retentate flow line configured to route retentate from the inlet side of the module to the inlet side of a subsequent module or otherwise discharge the retentate for recovery or recirculation within the system.
5. The system of paragraph 4, wherein the first and subsequent modules each have a permeate recycling line for returning permeate to the inlet side of the same module, and the modules subsequent to the first module further comprise a permeate recirculation line for backfeeding permeate to a preceding module within the system.
6. The system of paragraph 5, further comprising blending control for combining withdrawn permeate flows of two or more of the modules effective to provide a product having a targeted overall purity.
7. The system of paragraph 5, wherein the retentate flow line from each of the plurality of the modules is configured to route retentate from the retentate exit of a module to the inlet side of a next subsequent module.
8. The system of paragraph 5, wherein each filter comprises a membrane.
9. The system of paragraph 5, wherein the plurality of modules comprises 3 to 25 modules.
10. The system of paragraph 5, further comprising a control system to independently adjust one of product yield, product purity, net permeation rate, and overall flux while maintaining the three other variables approximately constant.
11. The system of paragraph 5, further comprising a plurality of pumps and valves for pumping and regulating flow of fluid through the system.
12. The system of paragraph 11, further comprising a plurality of sensors for acquiring data about fluid as it flows through the system, an electronic data processing network capable of at least receiving, transmitting, processing, and recording data associated with the operation of said pumps, valves, and sensors and wherein the recorded data collected during a flow filtration process is sufficiently comprehensive to allow control of the flow filtration process.
13. The system of paragraph 12, wherein the sensors are selected from at least one of flow rate sensors, pressure sensors, concentration sensors, pH sensors, conductivity sensors, temperature sensors, turbidity sensors, ultraviolet absorbance sensors, fluorescence sensors, refractive index sensors, osmolarity sensors, dried solids sensors, near infrared light sensors, or Fourier transform infrared light sensors.
14. The system of paragraph 4, wherein the first module includes a permeate recycling line for returning permeate to the inlet side of the first module, and wherein the modules subsequent to the first module each comprise a permeate recycling line for returning permeate to the inlet side of the same module and a permeate recirculation line for backfeeding permeate to a directly preceding module, and
   wherein the system further comprises:
   a first heat exchanger in thermal contact with the permeate withdrawal line of the first module,
   a second heat exchanger in thermal contact with at least one permeate recirculation line of the subsequent modules,
   a coolant supply line in fluid communication with at least one of the first and second heat exchangers.
15. The system of paragraph 4, wherein a permeate flow line of each of the subsequent modules after the first module comprises: i) a respective recirculation line backfeeding the inlet side of a directly preceding module within the system, and ii) a respective tap line for diverting permeate flow from the recirculation line for withdrawal from the system.
16. The system of paragraph 15, further comprising blending control for withdrawn permeate flows of the first module and one or more of the subsequent modules effective to provide a product having a targeted overall purity.
17. The system of paragraph 15, wherein the retentate flow line from the first module and at least one of the subsequent modules is configured to backfeed retentate from the retentate exit of a module to the inlet side of an immediate subsequent module within the system.
18. The system of paragraph 15, wherein each filter comprises a microfiltration membrane.
19. A filtration purification process comprising:
   a) introducing feed material to a first module comprising routing fresh feed to an inlet side of a first module of fluidly-interconnected filtration modules comprising the first module and multiple subsequent modules, and optionally introducing diluent at said inlet side,
   b) routing the feed material adjacent a filter in the first module to provide permeate and retentate,
   c) routing retentate from the first module, and optionally diluent, downstream to an inlet side of a subsequent module as subsequent module feed material to the subsequent module, for flow adjacent a filter to provide subsequent module retentate and subsequent module permeate,
   d) routing the subsequent module retentate, and optionally diluent, to a next subsequent module, for flow adjacent a filter to provide a next subsequent module retentate and a next subsequent module permeate,
   e) repeating step d) at least once;
   f) returning permeate in a plurality of the modules to an inlet side of a same module or backfeeding permeate to an upstream module,
   g) withdrawing permeate from at least one of the modules as a product material.
20. The process of paragraph 19, further comprising controlling independent adjustment of one of product yield, product purity, net permeation rate, and overall flux while maintaining the three other variables approximately constant.
21. The process of paragraph 19, comprising returning permeate in the first and subsequent modules to the inlet side of a same module, and backfeeding permeate in subsequent modules to the inlet side of an upstream module.
22. A filtration purification process comprising:
   a) introducing fresh feed to an inlet side of a first module of fiuidly-interconnected filtration modules comprising the first module and multiple subsequent modules, and optionally introducing diluent at said inlet side to provide a feed material,
   b) flowing the feed material adjacent a filter for selectively separating a species of interest from a mixture in the feed material for providing permeate that passes through the filter to an outlet side of the module and retentate that does not pass through the filter,
   c) directing permeate from the outlet side into a permeate flow line for discharging permeate from the first module and optionally recycling permeate to the inlet side of the first module to join with the feed material, and discharging retentate from the first module into a retentate flow line configured to route retentate to the inlet side of a subsequent module
   d) flowing the retentate via the retentate flow line of a preceding module to an inlet side of a subsequent module, optionally with diluent introduced at the inlet side thereof, and across a filter for selectively separating the species of interest from a mixture in feed material, providing permeate passing through the filter to an outlet side of the module and retentate that does not pass through the filter,
   e) routing permeate into a permeate flow line configured for returning permeate to at least the inlet side of the same subsequent module to form part of feed material thereto or for backfeeding permeate to a preceding module for combination with the respective feed material thereof, optionally routing permeate into a permeate withdrawal line, and directing retentate of the subsequent module to an inlet side of a next subsequent module within the system,
   f) repeating steps d) and e) at least once, and
   g) recovering product as an overall permeate comprising permeate withdrawn from at least one of the modules.
23. The process of paragraph 22, further comprising returning permeate via a permeate recycling line to the inlet side of the same module at each of said plurality of modules, and backfeeding permeate at each of the subsequent modules to an inlet side of a preceding module.
24. The process of paragraph 23, further comprising combining withdrawn permeate flows of two or more of the modules effective to provide a product having a targeted overall purity.
25. The process of paragraph 23, wherein the passing of the permeate through a filter at each module occurs through a microfiltration membrane.
26. The process of paragraph 23, further comprising independently adjusting one of product yield, product purity, net permeation rate, and overall flux while maintaining the three other variables approximately constant.
27. The process of paragraph 23, wherein fluid flow of feed material is maintained at a flux of about 0.1 to about 200 L/m²/hr/bar during tangential flow relative to a membrane filter in each module, and wherein each module is maintained under pressures of about 0.1 to about 60 bar.
28. The process of paragraph 23, wherein at least one of the separating steps is carried out at a temperature of from about 1 °C to about 75 °C.
29. The process of paragraph 23, wherein at least one of the separating steps is carried out at a pH of from about pH 2 to about pH 10.
30. The process of paragraph 23, wherein at least one of the filtration modules comprises at least one of a ceramic filter, a stainless steel filter, a hollow fiber filter, a tubular filter, a spiral filter, a flat sheet filter, or other filter configuration.
31. The process of paragraph 30, wherein the filter has a filter pore size of from about 0.005 micron to about 10 microns.
32. The process of paragraph 30, wherein the filter has a filter pore size of from about 0.05 micron to about 10 microns.
33. The process of paragraph 23, wherein a protein, a polypeptide, a nucleic acid, a glycoprotein, a biopolymer, or a small molecule is recovered.
34. The process of paragraph 23, wherein the feed from outside the system comprises a fermentation product of a bacterial production organism.
35. The process of paragraph 34, wherein the bacterial production organism is selected from the group consisting of Bacillus sp, Escherichia sp, Pantoea sp, Streptomyces sp, and Pseudomonas sp.
36. The process of paragraph 23, wherein the feed from outside the system comprises a fermentation product from a fungal production host.
37. The process of paragraph 36, wherein the fungal production host is selected from the group consisting of Aspergillus sp, Trichoderma sp, Schizosaccharomyces sp, Saccharomyces sp, Fusarium sp, Humicola sp, Mucor sp, Kluyveromyces sp, Yarrowia sp, Acremonium sp, Neurospora sp, Penicillium sp, Myceliophthora sp, and Thielavia sp.
38. The process of paragraph 23, wherein the retentate comprises a cell paste and the process further comprises collecting the cell paste.
39. The process of paragraph 23, wherein the process is a microfiltration or an ultrafiltration process.
40. A permeate product of the process of paragraph 19.
41. A permeate product of the process of paragraph 21.
42. A permeate product of a membrane separation of a feed material comprising an enzyme according to the process of paragraph 21, said product comprising an enzyme source having at least 4% greater activity defined by an increase in active to total protein ratio relative to a permeate product of a similar enzyme source obtained by rotary drum vacuum filtration.
43. A permeate product of a membrane separation of a feed material comprising an enzyme according to the process of paragraph 21, said product comprising an enzyme source having at least 10% greater activity defined by an increase in active to total protein ratio relative to a permeate product of a similar enzyme source obtained by rotary drum vacuum filtration.
44. A permeate product of a membrane separation of a feed material comprising an enzyme according to the process of paragraph 21, said product comprising an enzyme source having at least 40% lower color, wherein color is based on absorbance values measured at 470 and or 520nm using a spectrophotometer, relative to a permeate product of a similar enzyme source obtained by rotary drum vacuum filtration.
45. A permeate product of a membrane separation of a feed material comprising an enzyme according to the process of paragraph 21, said product comprising an enzyme source having at least 3% greater activity/nitrogen relative to a permeate product of a similar enzyme source obtained by rotary drum vacuum filtration.
46. A permeate product of a membrane separation of a feed material comprising an enzyme according to the process of paragraph 21, said product comprising an enzyme source having at least 5% greater activity/carbon relative to a permeate product of a similar enzyme source obtained by rotary drum vacuum filtration.
47. A retentate product of the process of paragraph 19.
48. The process of paragraph 22, further comprising:
   i) recycling permeate via a permeate recycling line to the inlet side of the first module,
   ii) withdrawing permeate from the system via a permeate withdrawal line from the outlet side of the first module,
   iii) returning permeate, at each of the subsequent modules, via a permeate recycling line to the inlet side of the same module,
   iv) backfeeding permeate, from each of the subsequent modules, via a permeate recirculation line to a directly preceding module within the system,
   v) thermally contacting a first heat exchanger with the permeate withdrawal line,
   vi) thermally contacting a second heat exchanger with at least one permeate recirculation line,
   vii) fluidly communicating a coolant via a coolant supply line with at least one of the first and second heat exchangers, and optionally introducing the coolant as diluent to one or more of the modules.
49. The process of paragraph 48, further comprising introducing cleaning chemical directly to an inlet side of each of the subsequent modules.
50. The process of paragraph 22, further comprising backfeeding permeate from each of the subsequent modules to the inlet sides of directly preceding modules via respective recirculation lines, and diverting permeate flow via one or more of respective tap lines associated with the recirculation lines for withdrawing permeate from the system.
51. The process of paragraph 50, further comprising combining withdrawn permeate flows of the first module and one or more of the subsequent modules effective to provide a product having a targeted overall purity.

## Claims

1. A permeate product of a filtration purification process, wherein the process comprises,
a) introducing feed material to a first module comprising routing fresh feed to an inlet side of a first module of fluidly-interconnected filtration modules comprising the first module and multiple subsequent modules, and optionally introducing diluent at the inlet side,
b) routing the feed material adjacent a filter in the first module to provide permeate and retentate,
c) routing retentate from the first module, and optionally diluent, downstream to an inlet side of a subsequent module as subsequent module feed material to the subsequent module, for flow adjacent a filter to provide subsequent module retentate and subsequent module permeate,
d) routing the subsequent module retentate, and optionally diluent, to a next subsequent module, for flow adjacent a filter to provide a next subsequent module retentate and a next subsequent module permeate,
e) repeating step d) at least once;
f) returning permeate in a plurality of the modules to an inlet side of a same module or backfeeding permeate to an upstream module; and
g) withdrawing permeate from at least one of the modules as a product material,
wherein the permeate product has an improved product quality as compared to products recovered using conventional industrial scale processes,
and wherein (i) permeate from at least one module is returned to the inlet side of the same module; and/or (ii) diafiltrate solution from multiple sources is introduced to the inlet side of each module.

2. The permeate product according to claim 1, wherein the purification process further comprises controlling independent adjustment of one of product yield, product purity, net permeation rate, and overall flux while maintaining the three other variables approximately constant.

3. The permeate product according to claim 1 or 2, wherein the purification process further comprises returning permeate in the first and subsequent modules to the inlet side of a same module, and backfeeding permeate in subsequent modules to the inlet side of an upstream module.

4. The permeate product according to any preceding claim, wherein the permeate product comprises an enzyme.

5. The permeate product according to claim 4, wherein the permeate product has at least 4% greater enzyme activity defined by an increase in active to total protein ratio relative to a permeate product of a similar feed material obtained by rotary drum vacuum filtration.

6. The permeate product according to claim 4, wherein the permeate product has at least 10% greater enzyme activity defined by an increase in active to total protein ratio relative to a permeate product of a similar feed material obtained by rotary drum vacuum filtration.

7. The permeate product according to claim 4, wherein the enzyme source has at least 40% lower color, wherein color is based on absorbance values measured at 470 and or 520nm using a spectrophotometer, relative to a permeate product of a similar enzyme source obtained by rotary drum vacuum filtration.

8. The permeate product according to claim 4, wherein the product comprises an enzyme source having at least 3% greater activity/nitrogen relative to a permeate product of a similar enzyme source obtained by rotary drum vacuum filtration.

9. The permeate product according to claim 4, wherein the product comprises an enzyme source having at least 5% greater activity/carbon relative to a permeate product of a similar enzyme source obtained by rotary drum vacuum filtration.

10. The permeate product according to claim 9, wherein the enzyme is selected from the group consisting of: phytases, xylanases, β-glucanases, phosphatases, proteases, amylases (alpha or beta), glucoamylases, cellulases, lipases, cutinases, oxidases, transferases, reductases, hemicellulases, mannanases, esterases, isomerases, pectinases, lactases, peroxidases, laccases, and mixtures thereof.
